(19) Europäisches Patentamt
European Patent Office
Office européen des brevets

(11) EP 3 581 578 A1

(12) **EUROPEAN PATENT APPLICATION**
published in accordance with Art. 153(4) EPC

(43) Date of publication:
**18.12.2019 Bulletin 2019/51**

(21) Application number: **18751525.9**

(22) Date of filing: **08.02.2018**

(51) Int Cl.:
***C07K 1/18** (2006.01)* ***B01D 15/36** (2006.01)*
***B01J 39/05** (2017.01)* ***B01J 39/26** (2006.01)*
***B01J 41/04** (2017.01)* ***B01J 41/20** (2006.01)*
***C07K 14/62** (2006.01)*

(86) International application number:
**PCT/JP2018/004494**

(87) International publication number:
**WO 2018/147393 (16.08.2018 Gazette 2018/33)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(30) Priority: **10.02.2017 JP 2017023336**
**10.02.2017 JP 2017023337**
**27.03.2017 JP 2017061971**
**22.06.2017 JP 2017122533**

(71) Applicant: **Mitsubishi Chemical Corporation**
**Tokyo 100-8251 (JP)**

(72) Inventors:
- **ANDO, Shingo**
  **Tokyo 100-8251 (JP)**
- **FUKUDA, Yoshito**
  **Tokyo 100-8251 (JP)**
- **ZHANG, Hua**
  **Tokyo 100-8251 (JP)**

(74) Representative: **Ter Meer Steinmeister & Partner**
**Patentanwälte mbB**
**Nymphenburger Straße 4**
**80335 München (DE)**

(54) **SEPARATING AGENT FOR HUMAN INSULIN PURIFICATION AND HUMAN INSULIN PURIFICATION METHOD**

(57) The present invention is related to a separating agent for the purification of human insulin, ensuring that human insulin can be recovered in high yield when isolating human insulin from a solution containing human insulin and a specific insulin under specific liquid chromatography separation conditions by using the separating agent.

EP 3 581 578 A1

## Description

### TECHNICAL FIELD

The present invention relates to a separating agent for the purification of human insulin and a method for purifying human insulin.

### BACKGROUND ART

The industrial production of human insulin uses a genetic engineering technique where a peptide (proinsulin, miniproinsulin) serving as an insulin precursor is synthesized in Escherichia coli or yeast into which a specific gene is introduced. In this technique, a desired insulin molecule can be obtained by cleaving a specific site in the insulin precursor through reaction using an enzyme such as trypsin, but unwanted cleavage occurs in parallel within an amino-acid sequence of insulin at the time of enzymatic reaction, and an insulin-like molecule in which one or more amino acid molecules are missing from the desired insulin is by-produced as an impurity. The insulin-like molecule as an impurity includes, for example, desB23-30 insulin and desB30 insulin.

In addition, other insulin-like impurities by-produced in the insulin production process include an impurity in which a part of amino acid molecules in the desired insulin sequence are varied, and examples thereof include A21 desamidoinsulin.

Conventionally, in a purification (impurity-removal) step in the insulin production process, desB30 insulin as one of by-product impurities has a structure in which only one molecule (threonine at position B30) of amino acid is missing from insulin. Accordingly, it is difficult to remove this impurity directly from the insulin, and an industrial technique therefor has not been established.

As a general technique for the purification of insulin, for example, separation by liquid chromatography such as reverse-phase chromatography or ion-exchange chromatography is used, but a method for efficiently separating desB30 insulin from insulin by liquid chromatography has not been found.

In addition, a method for efficiently separating A21 desamidoinsulin from insulin by liquid chromatography has also not been found.

Non-Patent Literature 1 describes a method for separating insulin/desB30 insulin by reverse-phase liquid chromatography.

Patent Literature 1 describes a method for separating recombinant insulin/desB30 insulin by ion-exchange liquid chromatography under high-pressure conditions.

### CITATION LIST

### PATENT LITERATURE

Patent Literature 1: JP-A-10-182700 (the term "JP-A" as used herein means an "unexamined published Japanese patent application")

### NON-PATENT LITERATURE

Non-Patent Literature 1: Kromasil (registered trademark) of Akzo Nobel Company, Homepage News May 2016 "Using EternityXT stationary phase for analysis and purification under reversed-phase chromatography" [searched on February 7, 2017], the Internet (https://www.kromasil.com/notes/?archive)

### SUMMARY OF INVENTION

### TECHNICAL PROBLEM

However, conventionally known methods are insufficient in providing high-purity human insulin (sometimes called regular insulin) by separating desB30 insulin in an industrial scale.

For example, the method described in Non-Patent Literature 1 is not suitable for an insulin purification step on an industrial scale, because the method is at the analytical scale or a special reagent such as perchlorate must be added to the chromatography mobile phase.

In the method described in Patent Literature 1, the object to be separated from desB30 insulin is recombinant insulin (insulin derivative), and this insulin differs in the amino acid sequence from normal human insulin and therefore, can be more easily separated from desB30 insulin. Accordingly, it is thought that this method cannot achieve separation

of human insulin/desB30 insulin, which are closer in amino acid sequence to each other and show similarity in the structure.

The present invention has been made in consideration of these conventional circumstances.

An object of a first embodiment of the present invention is to provide a separating agent capable of effectively separating human insulin and desB30 insulin in a purification step in the insulin production process, and a method for purifying human insulin by using the separating agent.

An object of a second embodiment of the present invention is to provide a separating agent capable of effectively separating human insulin and A21 desamidoinsulin in a purification step in the insulin production process, and a method for purifying human insulin by using the separating agent.

SOLUTION TO PROBLEM

As a result of intensive studies, the present inventors have found that human insulin (regular insulin) and desB30 insulin can be efficiently separated by ion-exchange chromatography using specific conditions, and have accomplished the first embodiment of the present invention.

In addition, as a result of intensive studies, the present inventors have found that human insulin (regular insulin) and A21 desamidoinsulin can be efficiently separated by reverse-phase chromatography using specific conditions, and have accomplished the second embodiment of the present invention.

More specifically, the gist of the present invention resides in the followings.

[1] A separating agent for purification of human insulin,
wherein at the time of isolating human insulin represented by the following formula (1) under the following liquid chromatography separation conditions by using the separating agent from a solution containing human insulin represented by the following formula (1) and desB30 insulin represented by the following formula (2), a recovery rate of human insulin represented by the following formula (1) is 70% or more when recovered in 99.5% purity:
(Liquid Chromatography Separation Conditions)

Column size: 150 mm×4.6 mm inner diameter
Eluent: water/pH adjusting agent/water-soluble organic solvent/salt
Flow velocity: 1 mL/min
Column temperature: 25°C
Measurement wavelength: UV 280 nm
Sample: a 100 mg/mL solution prepared from an insulin mixture containing 96 mass% of human insulin represented by the following formula (1) and 4 mass% of desB30 insulin represented by the following formula (2)
Sample injection volume: 100 μL

[Chem. 1]

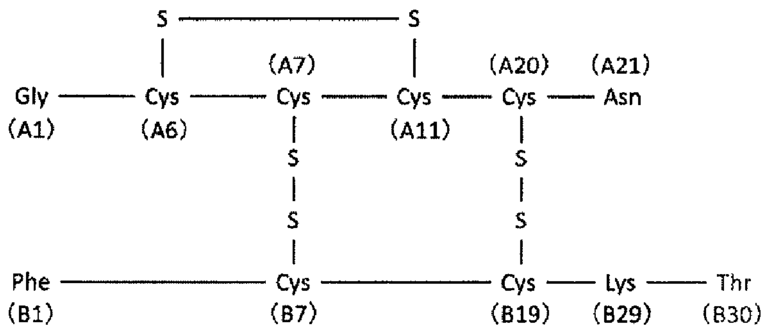

・・・(1)

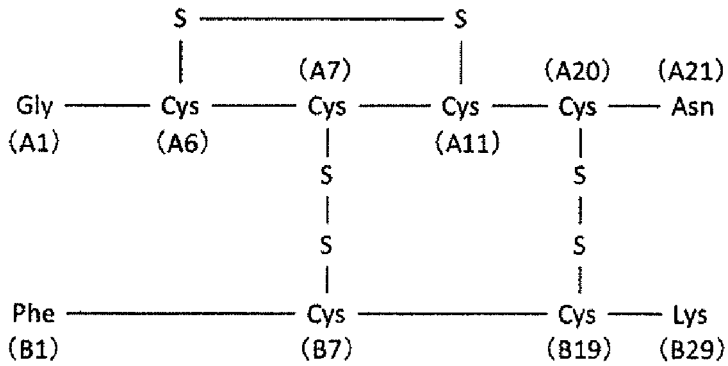

・・・(2) .

[2] The separating agent for purification of human insulin as described in [1], wherein a volume average particle diameter of the separating agent is from 1 to 50 μm.

[3] The separating agent for purification of human insulin as described in [1] or [2], containing: porous particles; and an ion exchange group bonded to the porous particles.

[4] The separating agent for purification of human insulin as described in [3], wherein the ion exchange group contains a strongly acidic functional group.

[5] The separating agent for purification of human insulin as described in [4], wherein an amount of the introduced strongly acidic functional group is from 0.045 to 0.25 meq/g per unit mass in a wet state of the separating agent.

[6] The separating agent for purification of human insulin as described in any one of [3] to [5], wherein the ion exchange group is a water-soluble polymer containing an ionic functional group.

[7] A separating agent for purification of human insulin, containing:

porous particles; and
an ion exchange group bonded to the porous particles, wherein
the ion exchange group contains a strongly acidic functional group, and
an amount of the introduced strongly acidic functional group is from 0.045 to 0.25 meq/g per unit mass in a wet state of the separating agent.

[8] A separating agent for purification of human insulin, wherein at the time of isolating human insulin represented by the following formula (1) under the following liquid chromatography separation conditions by using the separating agent from a solution containing human insulin represented by the following formula (1) and A21 desamidoinsulin represented by the following formula (3), a recovery rate of human insulin represented by the following formula (1) is 97% or more when recovered in 99.9% purity:
(Liquid Chromatography Separation Conditions)

Column size: 150 mm×4.6 mm inner diameter
Eluent: water/pH adjusting agent/water-soluble organic solvent

Flow velocity: 1 mL/min
Column temperature: 25°C
Measurement wavelength: UV 280 nm
Sample: a 100 mg/mL solution prepared from an insulin mixture containing 96 mass% of human insulin represented by the following formula (1) and 4 mass% of A21 desamidoinsulin represented by the following formula (3)
Sample injection volume: 400 μL

[Chem. 2]

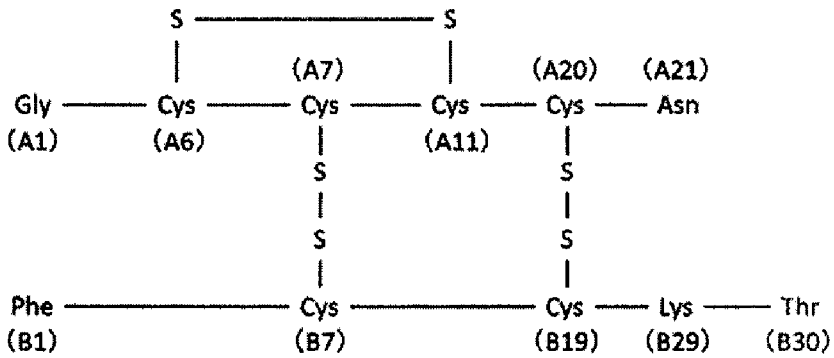

・・・(1)

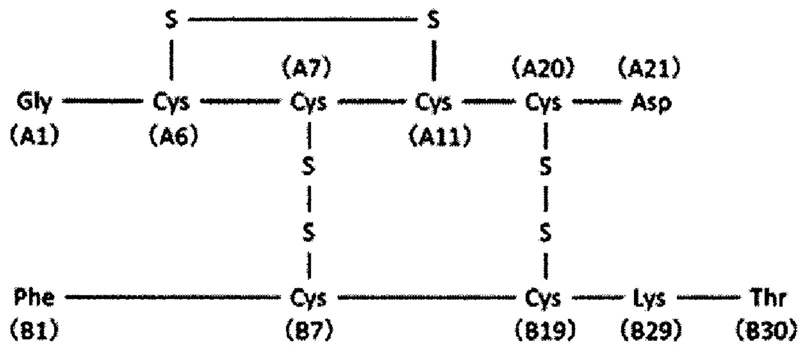

・・・(3)

[9] The separating agent for purification of human insulin as described in [8], wherein a volume average particle diameter of the separating agent is from 1 to 50 μm.

[10] The separating agent for purification of human insulin as described in [8] or [9], containing: porous particles; a hydrophobic functional group bonded to the porous particles; and an ion exchange group bonded to the porous particles.

[11] The separating agent for purification of human insulin as described in [10], wherein the hydrophobic functional group is at least one selected from the group consisting of a saturated alkyl group, an unsaturated alkyl group, and an aryl group.

[12] The separating agent for purification of human insulin as described in [10] or [11], wherein an amount of the introduced hydrophobic functional group is from 0.01 to 0.25 g/g per dry mass of the separating agent.

[13] The separating agent for purification of human insulin as described in any one of [10] to [12], wherein the ion exchange group is an anion exchange group.

[14] The separating agent for purification of human insulin as described in any one of [10] to [13], wherein an amount of the introduced ion exchange group is from 0.01 to 2.0 meq/g per dry mass of the separating agent.

[15] A separating agent for purification of human insulin, containing:

porous particles;
a hydrophobic functional group bonded to the porous particles; and
an ion exchange group bonded to the porous particles, wherein
the ion exchange group is an anion exchange group, and
an amount of the introduced ion exchange group is from 0.01 to 2.0 meq/g per dry mass of the separating agent.

[16] A human insulin purification method using the separating agent for purification of human insulin as described in any one of [1] to [15].

ADVANTAGEOUS EFFECTS OF INVENTION

By using the separating agent for the purification of human insulin according to the first embodiment of the present invention, desB30 insulin difficult to separate can be separated and removed, and the separating agent is industrially useful.

Furthermore, by using the human insulin purification method according to the first embodiment of the present invention, the load of a purification step in the conventional insulin production decreases, and reduction in the production constraints and cost can be expected.

In addition, by using the separating agent for the purification of human insulin according to the second embodiment of the present invention, A21 desamidoinsulin difficult to separate can be separated and removed, and the separating agent is industrially useful.

Furthermore, by using the human insulin purification method according to the second embodiment of the present invention, the load of a purification step in the conventional insulin production decreases, and reduction in the production constraints and cost can be expected.

DESCRIPTION OF EMBODIMENTS

The present invention is described in detail below, but the present invention is not limited to the following embodiments and can be implemented by making various changes within its gist. Here, in the present description, when values are expressed using "to" therebetween, the expression is used to include the values before and after "to".

Incidentally, in the present description, "(meth)acryl" indicates one or both of "acryl" and "methacryl", and the same holds true for "(meth)acrylate".

[First Embodiment]

<Separating Agent for Purification of Human Insulin>

The separating agent for the purification of human insulin according to the first embodiment of the present invention ensures that at the time of isolating human insulin represented by the following formula (1) under the following liquid chromatography separation conditions by using the separating agent from a solution containing human insulin represented by the following formula (1) and desB30 insulin represented by the following formula (2) (hereinafter, sometimes referred to as "insulin solution 1"), the recovery rate of human insulin represented by the following formula (1) is 70% or more when recovered in 99.5% purity.

Note that the "purity" and "recovery rate" can be calculated by the methods described in Examples.

(Liquid Chromatography Separation Conditions)

Column size: 150 mm×4.6 mm inner diameter
Eluent: water/pH adjusting agent/water-soluble organic solvent/salt
Flow velocity: 1 mL/min
Column temperature: 25°C
Measurement wavelength: UV 280 nm
Sample: a 100 mg/mL solution prepared from an insulin mixture containing 96 mass% of human insulin represented by the following formula (1) and 4 mass% of desB30 insulin represented by the following formula (2)
Sample injection volume: 100 μL

[Chem. 3]

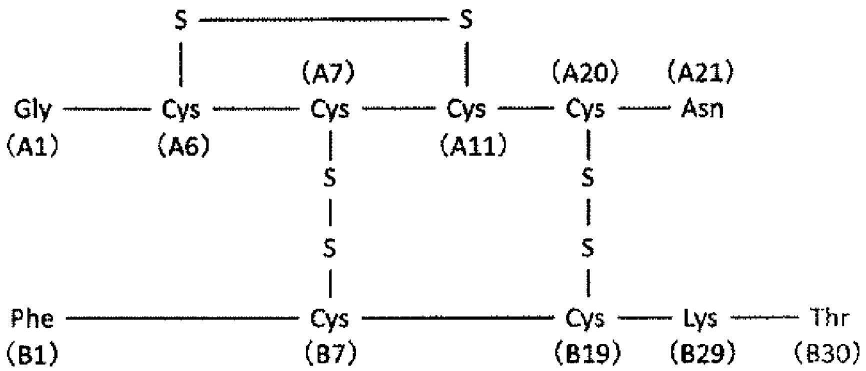

・・・(1)

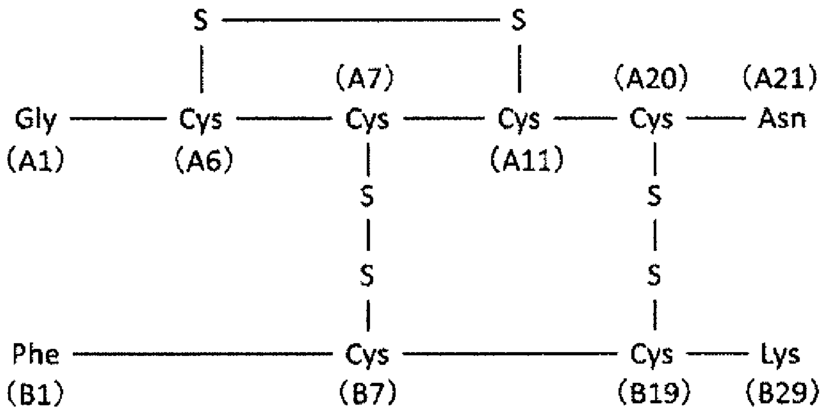

・・・(2)

[Insulin Solution 1]

The insulin solution 1 used in the first embodiment of the present invention is a solution containing human insulin represented by formula (1) and desB30 insulin represented by formula (2).

The method for obtaining the insulin solution 1 is not particularly limited but includes, for example, a method where an insulin precursor synthesized in Escherichia coli or yeast by a genetic engineering technique is cleaved through reaction using an enzyme to result in human insulin represented by formula (1).

At the time of the enzymatic reaction, usually, desB30 insulin represented by formula (2) in which only one molecule (threonine at position B30) of amino acid is missing from human insulin represented by formula (1) is by-produced. The amount of desB30 insulin by-produced may vary depending on the difference in the ratio of various enzymes used for the reaction, the reaction pH, the metal ion concentration in the reaction system, etc.

In the insulin solution 1, an impurity other than desB30 insulin may be contained. In addition, in the insulin solution 1, various substances used for the preparation and processing of human insulin and desB30 insulin may be contained.

The solvent in the insulin solution 1 may be sufficient if it sufficiently dissolves human insulin, and the solvent includes substances generally used in liquid chromatography, such as water, pH adjusting agent, organic solvent and salts. The solvent contained in the insulin solution 1 is preferably similar in composition and pH to the eluent but is not necessarily limited thereto.

[Porous Particles]

The separating agent for the purification of human insulin according to the first embodiment of the present invention preferably includes porous particles and an ion exchange group bonded to the porous particles.

The porous particles preferably used in the first embodiment of the present invention are not particularly limited as long as they are porous particles composed of a crosslinking synthetic polymer. The crosslinking synthetic polymer

includes, for example, a polyvinyl alcohol-based polymer, a styrene-divinylbenzene-based polymer, and a (meth)acrylic polymer, with a (meth)acrylic polymer being preferred.

Incidentally, in the present description, the styrene-divinylbenzene-based means that 50 mass% or more, preferably 80 mass% or more, of monomers as raw materials constituting the polymer are composed of styrene and divinylbenzene. In addition, in the present description, the (meth)acrylic means that 50 mass% or more, preferably 80 mass% or more, of monomers as raw materials constituting the polymer are composed of (meth)acrylate.

The spherical porous particles having a crosslinked structure, which is preferably used in the first embodiment of the present invention, is typically obtained by dispersing a monomer phase containing a polymerizable monovinyl monomer, a polymerizable polyvinyl monomer, a pore-forming agent, a polymerization initiator, etc. in an aqueous phase containing a dispersion stabilizer, etc., and performing a polymerization reaction by heating, etc. At this time, in order to bond the later-described ion exchange group to the porous particles, a reactive group is preferably present on the surface of the porous particles. The reactive group can be introduced by selecting, as the above-described polymerizable monovinyl monomer or polymerizable polyvinyl monomer, a monomer having a functional group capable of serving as a reactive group.

The porous particles preferably used in the first embodiment of the present invention are easily obtained through production utilizing a suspension polymerization method or a so-called seed polymerization method in which seed particles having a narrow particle dispersion (monodisperse particle size) are used and further impregnated with a vinyl monomer to bring about polymerization.

The reactive group includes, for example, a hydroxyl group, an amino group, a carboxyl group, a halogen group, an epoxy group, and a vinyl group, and in the case of preventing the reactive group itself from having ionicity, an epoxy group or a vinyl group is preferred.

The polymerizable monovinyl monomer used in the polymerization of the porous particles includes, for example, unsaturated carboxylic acids such as (meth)acrylic acid, itaconic acid and maleic acid; (meth)acrylic acid esters such as (meth)acrylic acid alkyl ester (e.g., methyl (meth)acrylate, ethyl (meth)acrylate), hydroxyethyl (meth)acrylate, hydroxypropyl (meth)acrylate, phenyl (meth)acrylate, 3-chloro-2-hydroxypropyl (meth)acrylate, 2-chloroethyl (meth)acrylate, glycidyl (meth)acrylate, 2,3-dihydroxypropyl (meth)acrylate, dimethylaminoethyl (meth)acrylate, diethylaminoethyl (meth)acrylate and tetrahydrofurfuryl (meth)acrylate; (meth)acrylamide derivatives such as (meth)acrylamide and N,N-dimethyl(meth)acrylamide; styrene and its alkyl or halogen substitutes, such as styrene, methylstyrene, ethylstyrene, $\alpha$-methylstyrene, chlorostyrene and chloromethylstyrene; vinyl esters such as vinyl acetate and vinyl propionate; vinyl ethers such as methyl vinyl ether and ethyl vinyl ether; allyl alcohol and its esters or ethers; and other vinyl compounds such as (meth)acrylonitrile, vinylsulfonic acid, p-styrenesulfonic acid, vinylpyridine and vinylpyrrolidone. These polymerizable monovinyl monomers need not be necessarily added and may be used individually or as a mixture of two or more types.

The polymerizable polyvinyl monomer includes, for example, aromatic polyvinyl compounds such as divinylbenzene, divinylnaphthalene and 2,4,6-trivinylethylbenzene; di(meth)acrylates such as (poly)ethylene glycol di(meth)acrylate, (poly)propylene glycol di(meth)acrylate, glycerol di(meth)acrylate, 1,4-butanediol di(meth)acrylate, neopentyl glycol di(meth)acrylate, tetramethylolmethane di(meth)acrylate and hydroquinone di(meth)acrylate; tri(meth)acrylates such as glycerol tri(meth)acrylate, trimethylolpropane tri(meth)acrylate and tetramethylolmethane tri(meth)acrylate; tetra(meth)acrylates such as tetramethylolmethane tetra(meth)acrylate and dipentaerythritol tetra(meth)acrylate; penta(meth)acrylates such as dipentaerythritol penta(meth)acrylate; hexa(meth)acrylates such as dipentaerythritol hexa(meth)acrylate; polycarboxylic acid polyvinyl esters and polycarboxylic acid polyallyl esters, such as divinyl adipate, diallyl maleate, diallyl phthalate and triallyl 1,3,5-benzenetricarboxylate; polyol polyvinyl ethers and polyol polyallyl ethers, such as divinyl ether, (poly)ethylene glycol divinyl ether, trimethylolpropane diallyl ether and pentaerythritol triallyl ether; and other polyvinyl compounds such as butadiene and methylenebisacrylamide. Of these, poly(meth)acrylates are preferred, and di(meth)acrylates are more preferred.

These polymerizable polyvinyl monomers may be used individually or as a mixture of two or more types. The ratio of the polymerizable polyvinyl monomer to all polymerizable vinyl monomers is from 30 to 100 mass%, preferably from 50 to 100 mass%. When the ratio of the polymerizable polyvinyl monomer to all polymerizable vinyl monomers is 30 mass% or more, the obtained polymeric particles have excellent mechanical strength.

The porous particles preferably used in the first embodiment of the present invention are favorably composed of a (meth)acrylic polymer and can be produced by using a known porous (meth)acrylic polymer as the base and introducing a desired functional group thereinto. For example, the porous particles include porous particles having a crosslinked structure, which is obtained by copolymerizing, as the polymerizable monovinyl monomer, a monovinyl(meth)acrylate having a functional group such as epoxy group, with a crosslinking (meth)acrylate as the polymerizable polyvinyl monomer. The functional groups contained in the monovinyl (meth)acrylate and crosslinking (meth)acrylate act as the reactive group of the porous particles.

The monovinyl (meth)acrylate having a functional group includes, for example, a (meth)acrylic acid polyhydric alcohol ester having one or more hydroxy groups, such as glycerol mono(meth)acrylate, 2-hydroxyethyl (meth)acrylate,

etc., an N-substituted (meth)acrylamide such as N-isopropylacrylamide, etc., acrylamide, methacrylamide, glycidyl (meth)acrylate, and a mixture thereof. In particular, glycerol mono(meth)acrylate, glycidyl (meth)acrylate, or a mixture thereof is preferred.

For example, in the case of imparting hydrophilicity to the porous particle surface, it is preferred that 5 mass% or more, more preferably 20 mass% or more, of monomers constituting the (meth)acrylic polymer are glycerol mono(meth)acrylate and/or glycidyl (meth)acrylate. In the case of using an epoxy group-containing monovinyl monomer such as glycidyl (meth)acrylate, hydrophilicity can be imparted to the porous particle surface by causing ring-opening of the epoxy group after the polymerization.

The crosslinking (meth)acrylate is preferably, for example, a poly(meth)acrylate or alkylene poly(meth)acrylamide of a polyhydric alcohol. Specifically, examples thereof include ethylene glycol di(meth)acrylate, polyethylene glycol di(meth)acrylate, glycerol di(meth)acrylate, allyl (meth)acrylate, trimethylolpropane tri(meth)acrylate, tetrahydroxybutane di(meth)acrylate, pentaerythritol di(meth)acrylate, methylenebisacrylamide, piperazine diacrylamide, and diallyltartardiamide, and these may be used individually or as a mixture. At least one selected from the group consisting of ethylene glycol di(meth)acrylate, glycerol di(meth)acrylate and trimethylolpropane tri(meth)acrylate is preferred.

For example, in the case of introducing a vinyl group as the reactive group into the porous particle surface, it is preferred that 50 mass% or more, more preferably 70 mass% or more, of monomers constituting the (meth)acrylic polymer are ethylene glycol di(meth)acrylate and/or trimethylolpropane tri(meth)acrylate. Furthermore, in the case of imparting hydrophilicity to the porous particle surface, it is preferred that 1 mass% or more, more preferably 5 mass% or more, of monomers constituting the (meth)acrylic polymer are glycerol di(meth)acrylate.

The porous particles preferably used in the first embodiment of the present invention can be made to have desired porosity by conducting the suspension polymerization or seed polymerization in the presence of a pore-forming agent.

The pore-forming agent, i.e., the pore-forming solvent is, for example, an organic solvent acting as a phase separation agent at the time of polymerization and promoting pore formation of the particles and dissolves the polymerizable vinyl monomer but does not dissolve the polymer thereof. Specifically, examples thereof include aliphatic and alicyclic hydrocarbons such as hexane, heptane, octane, dodecane and cyclohexane; aromatic hydrocarbons such as benzene, toluene, xylene and ethylbenzene; ketones such as methyl ethyl ketone; ethers such as dibutyl ether; aliphatic and alicyclic alcohols such as hexanol, octanol, dodecanol, cyclohexanol and lauryl alcohol; esters such as ethyl acetate, butyl acetate, dimethyl phthalate and diethyl phthalate; halogenated hydrocarbons such as dichloromethane, dichloroethane and trichloroethylene; aromatic halogenated hydrocarbons such as chlorobenzene and dichlorobenzene; etc. These solvents may be used individually or as a mixture of two or more types.

The amount added of the pore-forming solvent is from 50 to 400 mass%, preferably from 100 to 250 mass%, relative to all polymerizable vinyl monomers. When the amount added of the pore-forming solvent is not less than the lower limit value, pores for fully exerting the separation property in chromatography are sufficiently formed, and when the amount added of the pore-forming solvent is not more than the upper limit value, the obtained porous particles have excellent mechanical strength.

The polymerization initiator includes, for example, an azo-based polymerization initiator such as 2,2'-azobisisobutyronitrile, 2,2'-azobis(methyl isobutyrate), 2,2'-azobis(2,4-dimethylvaleronitrile) and 4,4-azobis(4-cyanopentanoic acid); a peroxide-based polymerization initiator such as tert-butyl hydroperoxide, di-tert-butyl peroxide, benzoyl peroxide, di-isopropyl peroxydicarbonate and tert-butyl peroxyisobutyrate; etc.

In addition, a chain transfer agent or a polymerization inhibitor may be appropriately added to the monomer phase for adjusting the polymerization reaction.

In the aqueous phase, a dispersion stabilizer is preferably added for increasing the dispersion stability of the monomer phase. The dispersion stabilizer includes, for example, known anionic and nonionic surfactants, and a synthetic polymer such as polyvinylpyrrolidone, polyethyleneimine and a vinyl alcohol-vinyl acetate copolymer.

Furthermore, a polymerization inhibitor or salts may be appropriately added to the aqueous phase, for example, for preventing aggregation of polymeric particles during polymerization.

The porous particles preferably used in the first embodiment of the present invention are favorably porous particles having at least one physical property out of the following 1) to 4), more preferably porous particles having at least two physical properties out of the following 1) to 4), still more preferably porous particles having at least three physical properties out of the following 1) to 4), yet still more preferably porous particles having all physical properties of the following 1) to 4).

1) The pore radius of porous particles is from 10 to 2,000 Å. The pore radius is more preferably 50 Å or more, still more preferably 100 Å or more, and is more preferably 1,000 Å or less, still more preferably 500 Å or less. When the pore radius is not less than the lower limit value, insulin molecules are readily diffused into the particle, and this advantageously raises the prospect of enhancing the separation performance or increasing the throughput. In addition, when the pore radius is not more than the upper limit value, this advantageously leads to an increase in

the mechanical strength of the separating agent and a decrease in the pressure loss during passing of liquid.

2) The pore volume is from 0.1 to 2.5 mL/g per unit mass of the porous particles. The pore volume is more preferably 0.4 mL/g or more, still more preferably 0.7 mL/g or more, and is more preferably 2.0 mL/g or less, still more preferably 1.5 mL/g or less. When the pore volume is not less than the lower limit value, insulin molecules are readily diffused into the particle, and this advantageously raises the prospect of enhancing the separation performance or increasing the throughput. In addition, when the pore volume is not more than the upper limit value, this advantageously leads to a decrease in the void ratio within the particles and an increase in the adsorption amount per column volume.

3) The specific surface area is from 10 to 1,000 $m^2/g$ per unit mass of the porous particle. The specific surface area is more preferably 50 $m^2/g$ or more, still more preferably 100 $m^2/g$ or more, and is more preferably 500 $m^2/g$ or less, still more preferably 300 $m^2/g$ or less. When the specific surface area is not less than the lower limit value, the frequency of interaction between the insulin molecule and the separating agent surface increases, and this advantageously raises the prospect of enhancing the separation performance or increasing the throughput. In addition, when the specific surface area is not more than the upper limit value, this advantageously makes it possible to decrease an unnecessary interaction giving rise to deterioration of the separation performance.

4) The volume average particle diameter of the porous particles is from 1 to 50 μm. The volume average particle diameter is more preferably from 3 to 20 μm, still more preferably from 5 to 14 μm. When the volume average particle diameter is not more than the upper limit value and is smaller, a high theoretical plate number can be obtained. On the other hand, when the volume average particle diameter is not less than the lower limit value, this is advantageous in that the pressure loss decreases and the problem associated with equipment is relieved to facilitate industrial use.

The volume average particle diameter, pore radius, pore volume and specific surface area of the separating agent for the purification of human insulin according to the first embodiment of the present invention are preferably within the same ranges as the characteristics of the above-described porous particles. Fundamentally, the particle diameter or pore properties of the separating agent for the purification of human insulin according to the first embodiment of the present invention reflect the characteristics of the porous particles used.

Incidentally, the volume average particle diameter is measured by the Coulter counter method. The sample is prepared as a dispersion with a predetermined aqueous sodium chloride solution, and an aperture for allowing the measured particle diameter to fall in the range of 2 to 60% of the aperture diameter is used. The measurement is performed by means of the Coulter counter using a suspension prepared by dispersing the sample in an aqueous 7 mass% sodium chloride solution such that the numerical value of the densitometer becomes from 5 to 10%. The average diameter of volume statistics obtained by the measurement is defined as the volume average particle diameter.

The separating agent for the purification of human insulin according to the first embodiment of the present invention having the above-described volume average particle diameter is obtained, for example, by a known classification method. As the classification method, for example, separation by a sieve, water separation using water flow, air separation using air flow, sedimentation classification using the difference in sedimentation rate, and centrifugal separation using the centrifugal force can be utilized. In addition, the separating agent for the purification of human insulin according to the first embodiment of the present invention having the above-described uniformity coefficient can also be obtained by the uniform particle size production technique described in Japanese Patent Application No. 63-116916 (JP-A-1-289802).

(Uniformity Coefficient)

The separating agent for the purification of human insulin according to the first embodiment of the present invention preferably has a sharp particle size distribution, and the uniformity coefficient represented by the following calculation method is usually 1.7 or less, preferably 1.5 or less, more preferably 1.3 or less. When the uniformity coefficient is not more than the upper limit value, at the time of packing porous crosslinked polymer particles into the column for reverse phase chromatography separation, the theoretical plate number of the column increases, and sufficient separation property is obtained.

[Uniformity Coefficient Calculation Method]

The uniformity coefficient can be determined from 25% diameter and 75% diameter of volume statics obtained by measurement, according to the following formula (I):

$$\text{Uniformity coefficient} = \{25\%\ \text{diameter}\ (\mu m)\}/\{75\%\ \text{diameter}\ (\mu m)\} \qquad (I)$$

[Ion Exchange Group]

The separating agent for the purification of human insulin according to the first embodiment of the present invention preferably includes porous particles and an ion exchange group bonded to the porous particles, as described above.

The ion exchange group is formed resulting from bonding of an ionic functional group to the porous particles. On this occasion, the ion exchange group may be bonded directly to the porous particles or may be bonded through a side chain having a structure other than an ionic functional group.

The ionic functional group preferably used in the first embodiment of the present invention includes, for example, a basic functional group such as primary amino group, secondary amino group, tertiary amino group and quaternary amino group, and an acidic functional group such as carboxyl group, sulfo group and phenol group. In order to achieve good separation when performing the purification at a pH lower than the isoelectric point (from 5.3 to 5.4) of human insulin, the ionic functional group is preferably an acidic functional group, more preferably a strongly acidic functional group, still more preferably a sulfo group.

On the other hand, in order to achieve good separation when performing the purification at a pH higher than the isoelectric point of human insulin, the ionic functional group is preferably a basic functional group.

The ion exchange group may be, as described above, an ionic functional group itself or may be a side chain including an ionic functional group and a structure other than an ionic functional group. Accordingly, the ion exchange group may be bonded directly to the porous particles or may be bonded through a side chain having a structure other than an ionic functional group.

The side chain including an ionic functional group and a structure other than an ionic functional group includes, for example, a sulfoalkyl group such as sulfopropyl group, an aromatic sulfo group such as sulfophenyl group, and a polymer containing a sulfo group-containing monomer such as 3-sulfopropyl methacrylate (and a salt thereof) and 2-acrylamido-2-methylpropanesulfonic acid (and a salt thereof).

The amount of the introduced ionic functional group typified by a strongly acidic functional group is preferably from 0.01 to 0.3 meq/g, more preferably from 0.045 to 0.25 meq/g, still more preferably from 0.1 to 0.2 meq/g, per unit mass of the separating agent in a wetted state with water.

Here, the wetted state as used in the present description indicates a state where separating agent particles dispersed in pure water and vacuum-filtered on a membrane filter (Hydrophilic Durapore SVLP04700 (pore size: 5 $\mu$m), manufactured by Merck Millipore) are recovered when water does not fall.

When the amount of the introduced ionic functional group typified by a strongly acidic functional group is not less than the lower limit value, insulin is likely to be sufficiently retained during chromatography, and the desired separation property can be achieved with ease. In addition, when the amount of the introduced ionic functional group is not more than the upper limit value, insulin is kept from being too strongly retained during chromatography, and this makes it easy to prevent elution from becoming difficult.

Furthermore, from the viewpoint of, in liquid chromatography separation, enhancing elution of insulin and preventing non-specific adsorption, the ion exchange group is preferably a water-soluble polymer containing an ionic functional group.

The water-soluble polymer containing an ionic functional group includes, for example, a polymer containing a sulfo group-containing monomer such as 3-sulfopropyl methacrylate and a salt thereof and 2-acrylamido-2-methylpropanesulfonic acid and a salt thereof, and a polymer formed by introducing an ionic functional group into a nonionic polymer such as dextran and pullulan.

As the method for introducing an ion exchange group into the porous particle surface, a known method can be used and, for example, the ion exchange group can be introduced by epoxy group that is the reactive group on the porous particle surface or reacting a known functionalizing agent via a functional group such as hydroxyl group and vinyl group. In the case of bonding a polymer compound as a side chain to the porous particle surface, a polymer compound prepared in advance may be bonded via a functional group on the porous particle surface, or preparation of a polymer compound and its bonding to a porous particle surface may be performed at the same time by surface graft polymerization, etc.

In the case of using a polymer compound as a side chain, its molecular weight or bonding amount to the porous particle surface may be appropriately selected, but as the molecular weight or bonding amount increases, when the separating agent is packed into the column, the pressure during passing of liquid rises. Therefore, the molecular weight or bonding amount is preferably selected within a range causing no practical problem.

In the separating agent for the purification of human insulin according to the first embodiment of the present invention, a side chain containing no ion exchange group and having hydrophilicity or hydrophobicity may be introduced so as to control the hydrophilicity/hydrophobicity of the porous particles. The hydrophilic functional group includes, for example, a hydroxyl group, an amide group and a cyano group, and the hydrophobic functional group includes, for example, an alkyl group such as octyl group and octadecyl group, and an aromatic group such as phenyl group and

benzyl group.

[Characteristic Features of Separating Agent for Purification of Human Insulin]

The separating agent for the purification of human insulin according to the first embodiment of the present invention is characterized in that at the time of isolating human insulin under the following liquid chromatography conditions, the recovery rate of human insulin is 70% or more when recovered in 99.5% purity.

In view of purification efficiency, the recovery rate of human insulin is preferably higher and is preferably 80% or more, more preferably 90% or more.

(Liquid Chromatography Separation Conditions)

Column size: 150 mm×4.6 mm inner diameter
Eluent: water/pH adjusting agent/water-soluble organic solvent/salt
Flow velocity: 1 mL/min
Column temperature: 25°C
Measurement wavelength: UV 280 nm
Sample: a 100 mg/mL solution prepared from an insulin mixture containing 96 mass% of human insulin represented by the following formula (1) and 4 mass% of desB30 insulin represented by the following formula (2)
Sample injection volume: 100 μL

[Chem. 4]

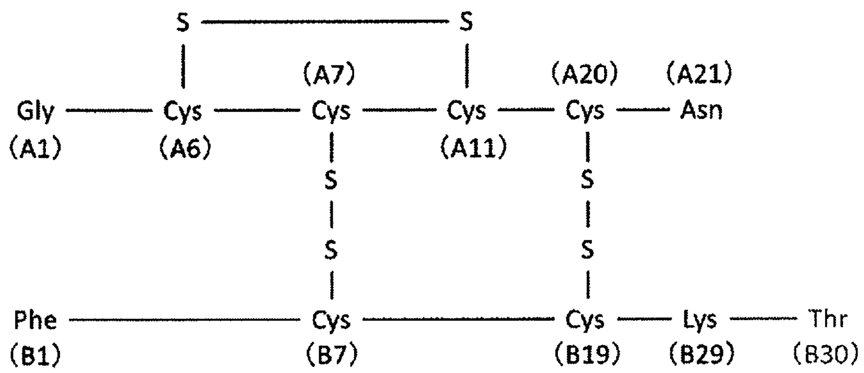

・・・(1)

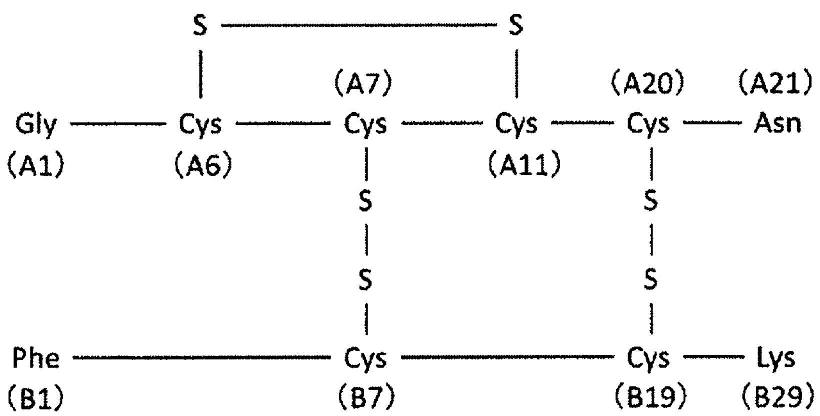

・・・(2)

The value of the recovery rate above can be appropriately adjusted by the volume average particle diameter,

pore properties, amount of surface functional group and combination of functional group species in the separating agent used, and the organic solvent concentration and pH of the mobile phase in liquid chromatography.

The eluent for liquid chromatography may be sufficient if it fully dissolves human insulin, and the eluent contains substances generally used in liquid chromatography, such as water, pH adjusting agent, water-soluble organic solvent and salt. The eluent is preferably similar in composition and pH to the solvent contained in the insulin solution 1 but is not necessarily limited thereto.

As the water-soluble organic solvent, for example, alcohols, acetonitrile, etc. are used and for obtaining high separation property and reducing the pressure during passing of liquid, acetonitrile is preferably used.

The water-soluble organic solvent concentration in the eluent is preferably from 5 to 50 vol%, more preferably 10 vol% or more, still more preferably 20 vol% or more, and is more preferably 40 vol% or less. When the water-soluble organic solvent concentration is 5 vol% or more, elution of insulin is facilitated, and when the concentration is 50 vol% or less, precipitation of salts added to the eluent can be suppressed.

As the eluent for liquid chromatography used in the first embodiment of the present invention, an eluent containing from 10 to 50 vol% of acetonitrile is particularly preferred.

In addition, the content of water in the eluent is preferably from 40 to 95 vol%, more preferably from 50 to 90 vol%, still more preferably from 60 to 80 vol%, in view of solubility of insulin, pH adjusting agent, etc.

Furthermore, the content of the pH adjusting agent in the eluent is preferably 0.01 mol/L or more per volume of the eluent so as to obtain sufficient pH adjusting ability and is preferably 1 mol/L or less so as not to inhibit interaction between the separating agent and insulin in chromatography.

As the pH adjusting agent, an acid, a base, and salts having a pH buffering ability are appropriately used. The pH adjusting agent includes, for example, an inorganic acid such as hydrochloric acid and phosphoric acid; salts of these inorganic acids; an organic acid such as acetic acid, citric acid and trifluoroacetic acid; and salts of these organic acids. An organic acid and an organic acid salt are preferred, and acetic acid is more preferred.

The pH of the eluent is preferably 5.0 or less or 6.0 or more, more preferably 4.0 or less or 7.0 or more. When the pH of the eluent is 5.0 or less or 6.0 or more, sufficient insulin solubility is achieved, and a trouble such as precipitation during purification can be suppressed.

The pH of the eluent in the elution step is preferably set according to the type of the separating agent used in liquid chromatography, particularly, the type of the ion exchange group. For example, the eluent is preferably set to a pH lower than the isoelectric point (from 5.3 to 5.4) of the target insulin to be purified in the case of using a separating agent into which an acidic functional group is introduced as the ion exchange group, and set to a pH higher than the isoelectric point of the target insulin to be purified in the case of using a separation agent into which a basic functional group is introduced as the ion exchange group.

The salt in the eluent is added for the purpose of allowing insulin adsorbed mainly to the separating agent to be eluted by the ion exchange action. The salt includes, for example, an inorganic salt such as sodium chloride, sodium sulfate and ammonium sulfate; and an organic salt such as sodium acetate and ammonium acetate, and the salt is appropriately selected in consideration of the material of purification equipment and the solubility, etc. in the eluent. For the reason that the cost is low in an industrial-scale application and the toxicity and hazardousness as a chemical substance are low, the salt in the eluent is preferably a salt except for perchloric acid and perchlorate.

The salt concentration in the eluent may appropriately vary in the elution step but, in view of preventing salt precipitation, is preferably 2 mol/L or less, more preferably 1 mol/L or less.

<Human Insulin Purification Method>

The purification method according to the first embodiment of the present invention is characterized by using the separating agent for the purification of human insulin according to the first embodiment of the present invention.

The purification method according to the first embodiment of the present invention is conducted by liquid chromatography using a separating agent-packed column and includes a step of preparing the above-described insulin solution 1, a step of preparing a separating agent-packed column, a loading step to the column, and a step of passing an eluent through the column to elute respective components. The purification method according to the first embodiment of the present invention is described below for each step.

[Preparation Step of Insulin Solution 1]

The insulin solution 1 used in the purification method according to the first embodiment of the present invention is the same as the insulin solution 1 described in

<Separating Agent for Purification of Human Insulin>.

In the purification method according to the first embodiment of the present invention, human insulin can be isolated by separating and removing mainly desB30 insulin from the insulin solution 1.

[Preparation Step of Separating Agent-Packed Column]

The purification method according to the first embodiment of the present invention is conducted by liquid chromatography using the separating agent for the purification of human insulin according to the first embodiment of the present invention.

Therefore, it is required to prepare the separating agent and pack the separating agent into the column.

In the purification method according to the first embodiment of the present invention, the separating agent is used in the state of being packed into the packing column for liquid chromatography, and the structure and size of the column are not particularly limited. The method for packing the separating agent into the column is also not particularly limited, but in order to form a uniform packed bed, wet packing of packing the separating agent into the column by charging it in a slurry state after dispersion in a packing solvent is preferred.

[Loading Step to Separating Agent-Packed Column]

In the purification method according to the first embodiment of the present invention, the insulin solution 1 is loaded to the column packed with the separating agent for the purification of human insulin according to the first embodiment of the present invention, and the purification is conducted by liquid chromatography.

In the step of loading the insulin solution 1 to the column, human insulin and desB30 insulin are retained within the column due to interaction of the separating agent with human insulin and desB30 insulin. In this step, an impurity that does not interact with the separating agent and is not retained on the column, exits the column and is thereby removed.

The insulin solution 1 loaded is preferably prepared to make human insulin and desB30 insulin be sufficiently dissolved and contains substances generally used in liquid chromatography, such as water, pH adjusting agent, organic solvent and salts. The solvent contained in the insulin solution 1 is preferably similar in composition to the above-described eluent but is not necessarily limited thereto.

As the organic solvent, for example, a water-soluble organic solvent such as alcohols and acetonitrile is used and for obtaining high separation property and reducing the pressure during passing of liquid, acetonitrile is preferably used.

The organic solvent concentration in the insulin solution 1 is preferably 50 vol% or less, more preferably 30 vol% or less, so as to prevent unnecessary elution of insulin in the loading step. In addition, as for the pH of the insulin solution 1, in order to prevent insulin precipitation in the loading step and pH variation within the column in the subsequent elution step, the pH difference from the above-described eluent is preferably 1 or less, more preferably 0.5 or less.

The total concentration of human insulin concentration and desB30 insulin concentration in the insulin solution 1 may be appropriately selected depending on the purpose of purification but in view of insulin solubility and separation efficiency, the total concentration is preferably 1,000 mg/mL or less, more preferably 500 mg/mL or less, still more preferably 100 mg/mL or less, and in order to obtain sufficient purification efficiency, is preferably 0.1 mg/mL or more, more preferably 1 mg/mL or more. The total concentration of human insulin concentration and desB30 insulin concentration in the insulin solution 1 can be adjusted by the amount of water, organic solvent or other additives such as pH adjusting agent.

As the pH adjusting agent, an acid, a base, and salts having a pH buffering ability are appropriately used. The pH adjusting agent includes, for example, an inorganic acid such as hydrochloric acid and phosphoric acid; salts of these inorganic acids; an organic acid such as acetic acid, citric acid and trifluoroacetic acid; and salts of these organic acids. An organic acid and an organic acid salt are preferred, and acetic acid is more preferred.

The pH of the insulin solution 1 after preparation is preferably 5.0 or less or 6.0 or more, more preferably 4.0 or less or 7.0 or more. When the pH of the insulin solution 1 is 5.0 or less or 6.0 or more, sufficient insulin solubility is achieved, and a trouble such as precipitation during purification can be suppressed.

In order to prevent unnecessary elution of insulin in the loading step, the salt concentration in the insulin solution 1 is preferably 0.5 mol/L or less, more preferably 0.2 mol/L or less.

The concentration of desB30 insulin contained in the insulin solution 1 is not particularly limited, but for the reason that when the amount of desB30 insulin is not more than a given amount relative to the amount of human insulin, the purification efficiency increases, the amount is preferably 50 mass% or less, more preferably 10 mass% or less, relative to the total amount of human insulin and desB30 insulin.

In addition, the amount of insulin loaded to the separating agent-packed column in one purification may be appropriately selected depending on the purpose of purification, but since when the amount loaded is not more than a

given amount, the purification efficiency increases, the insulin is loaded in an amount of preferably 100 mg/mL or less, more preferably 50 mg/mL or less, relative to the volume of the separating agent packed into the column.

Before and after the loading of the insulin solution 1 to the column, if desired, the interior of the column is washed and equilibrated with a washing solution having the same or similar composition to the eluent used in the elution step. The organic solvent concentration in the washing solution is preferably not more than the concentration of the organic solvent contained in the eluent so as to prevent unnecessary acceleration of the elution of insulin after loading. In addition, as for the pH of the washing solution, in order to prevent insulin precipitation after loading or pH variation within the column in the elution step, the pH difference from the above-described eluent is preferably 1 or less, more preferably 0.5 or less.

[Elution Step]

The purification method according to the first embodiment of the present invention includes a step of passing the above-described eluent through the insulin solution 1-loaded column to elute human insulin and perform elution of respective components. In particular, it is preferable to include a step of eluting human insulin with a salt-containing eluent, and in this case, gradient elution in which the salt concentration in the eluent is changed over time, and isocratic elution in which the salt concentration is constant, are used.

The separating agent for the purification of human insulin according to the first embodiment of the present invention can be suitably used for the purification of human insulin, because human insulin and desB30 insulin can be effectively separated.

The purification above can be performed by packing the separating agent for the purification of human insulin according to the first embodiment of the present invention into a column, and passing a human insulin solution containing desB30 insulin as an impurity through the column.

[Second Embodiment]

<Separating Agent for Purification of Human Insulin>

The separating agent for the purification of human insulin according to the second embodiment of the present invention ensures that at the time of isolating human insulin by use of the separating agent under the following liquid chromatography separation conditions from a solution containing human insulin represented by the following formula (1) and A21 desamidoinsulin represented by the following formula (3) (hereinafter, sometimes referred to as "insulin solution 2"), the recovery rate of human insulin is 97% or more when recovered in 99.9% purity.

Note that the "purity" and "recovery rate" can be calculated by the methods described in Examples.

(Liquid Chromatography Separation Conditions)

Column size: 150 mm×4.6 mm inner diameter
Eluent: water/pH adjusting agent/water-soluble organic solvent
Flow velocity: 1 mL/min
Column temperature: 25°C
Measurement wavelength: UV 280 nm
Sample: a 100 mg/mL solution prepared from an insulin mixture containing 96 mass% of human insulin represented by the following formula (1) and 4 mass% of A21 desamidoinsulin represented by the following formula (3)
Sample injection volume: 400 μL

[Chem. 5]

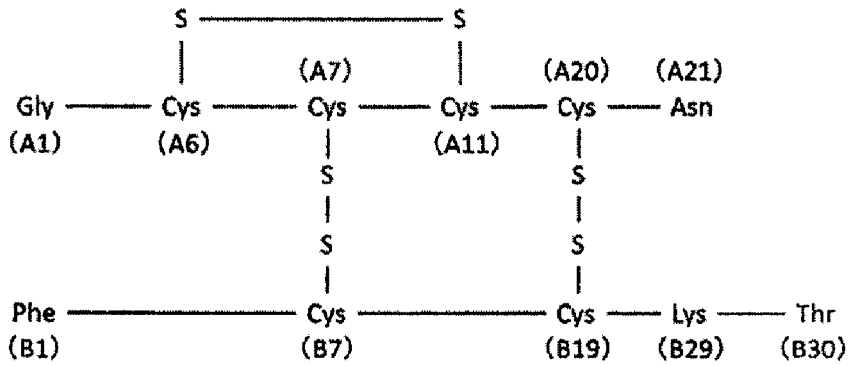

・・・(1)

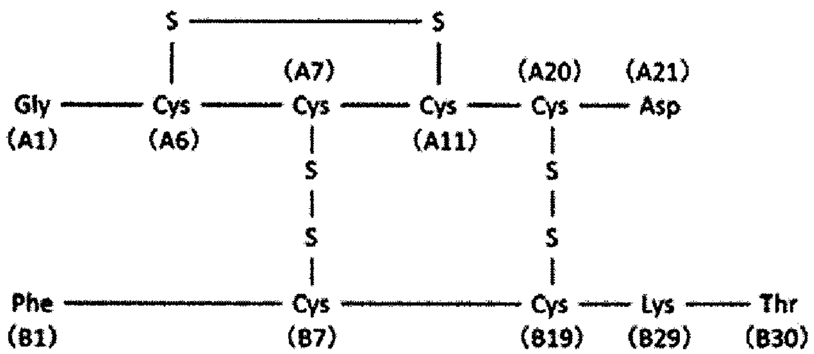

・・・(3)

[Insulin Solution 2]

The insulin solution 2 used in the second embodiment of the present invention is a solution containing human insulin represented by formula (1) and A21 desamidoinsulin represented by formula (3).

The A21 desamidoinsulin represented by formula (3) is produced as an impurity, for example, when human insulin represented by formula (1) is held in an acidic solution.

In the insulin solution 2, an impurity other than A21 desamidoinsulin may be contained. In addition, in the insulin solution 2, various substances used for the preparation and processing of human insulin and A21 desamidoinsulin may be contained.

The solvent in the insulin solution 2 may be sufficient if it sufficiently dissolves human insulin, and the solvent includes substances generally used in liquid chromatography, such as water, pH adjusting agent, organic solvent and salts. The solvent contained in the insulin solution 2 is preferably similar in composition and pH to the eluent but is not necessarily limited thereto.

[Porous Particles]

The separating agent for the purification of human insulin according to the second embodiment of the present invention preferably includes porous particles, a hydrophobic functional group bonded to the porous particles, and an ion exchange group bonded to the porous particles.

The porous particles preferably used in the second embodiment of the present invention are not particularly limited as long as they are porous particles composed of a crosslinking synthetic polymer. The crosslinking synthetic polymer includes, for example, a polyvinyl alcohol-based polymer, a styrene-divinylbenzene-based polymer, and a (meth)acrylic polymer, with a (meth)acrylic polymer being preferred.

Incidentally, in the present description, the styrene-divinylbenzene-based means that 50 mass% or more,

preferably 80 mass% or more, of monomers as raw materials constituting the polymer are composed of styrene and divinylbenzene. In addition, in the present description, the (meth)acrylic means that 50 mass% or more, preferably 80 mass% or more, of monomers as raw materials constituting the polymer are composed of (meth)acrylate.

The spherical porous particles having a crosslinked structure, which is preferably used in the second embodiment of the present invention, is typically obtained by dispersing a monomer phase containing a polymerizable monovinyl monomer, a polymerizable polyvinyl monomer, a pore-forming agent, a polymerization initiator, etc. in an aqueous phase containing a dispersion stabilizer, etc., and performing a polymerization reaction by heating, etc. At this time, in order to bond the later-described hydrophobic functional group and ion exchange group to the porous particle surface, a reactive group needs to be present on the surface of the porous particles. The reactive group can be introduced by selecting, as the above-described polymerizable monovinyl monomer or polymerizable polyvinyl monomer, a monomer having a functional group capable of serving as a reactive group.

The porous particles preferably used in the second embodiment of the present invention are easily obtained through production utilizing a suspension polymerization method or a so-called seed polymerization method in which seed particles having a narrow particle dispersion (monodisperse particle size) are used and further impregnated with a vinyl monomer to bring about polymerization.

The reactive group includes, for example, a hydroxyl group, an amino group, a carboxyl group, a halogen group, an epoxy group, and a vinyl group, and in view of ease of introduction of the functional group, a vinyl group and an epoxy group are preferred.

The polymerizable monovinyl monomer used in the polymerization of the porous particles includes, for example, unsaturated carboxylic acids such as (meth)acrylic acid, itaconic acid and maleic acid; (meth)acrylic acid esters such as (meth)acrylic acid alkyl ester (e.g., methyl (meth)acrylate, ethyl (meth)acrylate), hydroxyethyl (meth)acrylate, hydroxypropyl (meth)acrylate, phenyl (meth)acrylate, 3-chloro-2-hydroxypropyl (meth)acrylate, 2-chloroethyl (meth)acrylate, glycidyl (meth)acrylate, 2,3-dihydroxypropyl (meth)acrylate, dimethylaminoethyl (meth)acrylate, diethylaminoethyl (meth)acrylate and tetrahydrofurfuryl (meth)acrylate; (meth)acrylamide derivatives such as (meth)acrylamide and N,N-dimethyl(meth)acrylamide; styrene and its alkyl or halogen substitutes, such as styrene, methylstyrene, ethylstyrene, $\alpha$-methylstyrene, chlorostyrene and chloromethylstyrene; vinyl esters such as vinyl acetate and vinyl propionate; vinyl ethers such as methyl vinyl ether and ethyl vinyl ether; allyl alcohol and its esters or ethers; and other vinyl compounds such as (meth)acrylonitrile, vinylsulfonic acid, p-styrenesulfonic acid, vinylpyridine and vinylpyrrolidone. These polymerizable monovinyl monomers need not be necessarily added and may be used individually or as a mixture of two or more types.

The polymerizable polyvinyl monomer includes, for example, aromatic polyvinyl compounds such as divinylbenzene, divinylnaphthalene and 2,4,6-trivinylethylbenzene; di(meth)acrylates such as (poly)ethylene glycol di(meth)acrylate, (poly)propylene glycol di(meth)acrylate, glycerol di(meth)acrylate, 1,4-butanediol di(meth)acrylate, neopentyl glycol di(meth)acrylate, tetramethylolmethane di(meth)acrylate and hydroquinone di(meth)acrylate; tri(meth)acrylates such as glycerol tri(meth)acrylate, trimethylolpropane tri(meth)acrylate and tetramethylolmethane tri(meth)acrylate; tetra(meth)acrylates such as tetramethylolmethane tetra(meth)acrylate and dipentaerythritol tetra(meth)acrylate; penta(meth)acrylates such as dipentaerythritol penta(meth)acrylate; hexa(meth)acrylates such as dipentaerythritol hexa(meth)acrylate; polycarboxylic acid polyvinyl esters and polycarboxylic acid polyallyl esters, such as divinyl adipate, diallyl maleate, diallyl phthalate and triallyl 1,3,5-benzenetricarboxylate; polyol polyvinyl ethers and polyol polyallyl ethers, such as divinyl ether, (poly)ethylene glycol divinyl ether, trimethylolpropane diallyl ether and pentaerythritol triallyl ether; and other polyvinyl compounds such as butadiene and methylenebisacrylamide. Of these, poly(meth)acrylates are preferred, and di(meth)acrylates are more preferred.

These polymerizable polyvinyl monomers may be used individually or as a mixture of two or more types. The ratio of the polymerizable polyvinyl monomer to all polymerizable vinyl monomers is from 30 to 100 mass%, preferably from 50 to 100 mass%. When the ratio of the polymerizable polyvinyl monomer to all polymerizable vinyl monomers is 30 mass% or more, the obtained porous particles have excellent mechanical strength.

The porous particles preferably used in the second embodiment of the present invention are favorably composed of a (meth)acrylic polymer and can be produced by using a known porous (meth)acrylic polymer as the base and introducing a desired functional group thereinto. For example, the porous particles include porous particles having a crosslinked structure, which is obtained by copolymerizing, as the polymerizable monovinyl monomer, a monovinyl(meth)acrylate having a functional group such as epoxy group, with a crosslinking (meth)acrylate as the polymerizable polyvinyl monomer. The functional groups contained in the monovinyl (meth)acrylate and crosslinking (meth)acrylate act as the reactive group of the porous particles.

The monovinyl (meth)acrylate having a functional group includes, for example, a (meth)acrylic acid polyhydric alcohol ester having one or more hydroxy groups, such as glycerol mono(meth)acrylate, 2-hydroxyethyl (meth)acrylate, etc., an N-substituted (meth)acrylamide such as N-isopropylacrylamide, etc., acrylamide, methacrylamide, glycidyl (meth)acrylate, and a mixture thereof. In particular, glycerol mono(meth)acrylate, glycidyl (meth)acrylate, or a mixture thereof is preferred.

For example, for the reason that a functional group necessary for exhibiting sufficient separation performance needs to be introduced, preferably 10 mass% or more, more preferably 20 mass% or more, of monomers constituting the (meth)acrylic polymer are glycidyl (meth)acrylate.

The crosslinking (meth)acrylate is preferably, for example, a poly(meth)acrylate or alkylene poly(meth)acrylamide of a polyhydric alcohol. Specifically, examples thereof include ethylene glycol di(meth)acrylate, polyethylene glycol di(meth)acrylate, glycerol di(meth)acrylate, allyl (meth)acrylate, trimethylolpropane tri(meth)acrylate, tetrahydroxybutane di(meth)acrylate, pentaerythritol di(meth)acrylate, methylenebisacrylamide, piperazine diacrylamide, and diallyltartardiamide, and these may be used individually or as a mixture. At least one selected from the group consisting of ethylene glycol di(meth)acrylate, glycerol di(meth)acrylate and trimethylolpropane tri(meth)acrylate is preferred.

For example, for the reason that the mechanical strength of the porous particles is maintained or the pressure resistance during passing of liquid is ensured, preferably 30 mass% or more, more preferably 50 mass% or more, of monomers constituting the (meth)acrylic polymer are ethylene glycol di(meth)acrylate.

The ratio of monovinyl (meth)acrylate and crosslinking (meth)acrylate for satisfying both the amount of the introduced functional group necessary to exert high separation performance and the good liquid-passing performance due to low pressure loss is preferably monovinyl (meth)acrylate/crosslinking (meth)acrylate (ratio by mass) = from 70/30 to 0/100, more preferably from 30/70 to 10/90.

The porous particles preferably used in the second embodiment of the present invention can be made to have desired porosity by conducting the suspension polymerization or seed polymerization in the presence of a pore-forming agent.

The pore-forming agent, i.e., the pore-forming solvent is an organic solvent acting as a phase separation agent at the time of polymerization and promoting pore formation of the particles and dissolves the polymerizable vinyl monomer but does not dissolve the polymer thereof. Specifically, examples thereof include aliphatic and alicyclic hydrocarbons such as hexane, heptane, octane, dodecane and cyclohexane; aromatic hydrocarbons such as benzene, toluene, xylene and ethylbenzene; ketones such as methyl ethyl ketone; ethers such as dibutyl ether; aliphatic and alicyclic alcohols such as hexanol, octanol, dodecanol, cyclohexanol and lauryl alcohol; esters such as ethyl acetate, butyl acetate, dimethyl phthalate and diethyl phthalate; halogenated hydrocarbons such as dichloromethane, dichloroethane and trichloroethylene; aromatic halogenated hydrocarbons such as chlorobenzene and dichlorobenzene; etc. These solvents may be used individually or as a mixture of two or more types.

The amount added of the pore-forming solvent is from 50 to 400 mass%, preferably from 100 to 250 mass%, relative to all polymerizable vinyl monomers. When the amount added of the pore-forming solvent is not less than the lower limit value, pores for fully exerting the separation property in chromatography are sufficiently formed, and when the amount added of the pore-forming solvent is not more than the upper limit value, the obtained porous particles have excellent mechanical strength.

The polymerization initiator includes, for example, an azo-based polymerization initiator such as 2,2'-azobisisobutyronitrile, 2,2'-azobis(methyl isobutyrate), 2,2'-azobis(2,4-dimethylvaleronitrile) and 4,4-azobis(4-cyanopentanoic acid); a peroxide-based polymerization initiator such as tert-butyl hydroperoxide, di-tert-butyl peroxide, benzoyl peroxide, di-isopropyl peroxydicarbonate and tert-butyl peroxyisobutyrate; etc.

In addition, a chain transfer agent or a polymerization inhibitor may be appropriately added to the monomer phase for adjusting the polymerization reaction.

In the aqueous phase, a dispersion stabilizer is preferably added for increasing the dispersion stability of the monomer phase. The dispersion stabilizer includes, for example, known anionic and nonionic surfactants, and a synthetic polymer such as polyvinylpyrrolidone, polyethyleneimine and a vinyl alcohol-vinyl acetate copolymer.

Furthermore, a polymerization inhibitor, a suspension stabilizer or salts may be appropriately added to the aqueous phase, for example, for preventing aggregation of polymeric particles during polymerization.

The porous particles preferably used in the second embodiment of the present invention are favorably porous particles having at least one physical property out of the following 1) to 4), more preferably porous particles having at least two physical properties out of the following 1) to 4), still more preferably porous particles having at least three physical properties out of the following 1) to 4), yet still more preferably porous particles having all physical properties of the following 1) to 4).

1) The pore radius of porous particles is from 10 to 1,000 Å. The pore radius is more preferably 50 Å or more, still more preferably 150 Å or more, and is more preferably 500 Å or less, still more preferably 300 Å or less. When the pore radius is not less than the lower limit value, high molecules such as human insulin or protein are readily diffused into the particles, and this advantageously raises the prospect of enhancing the separation performance or increasing the throughput. In addition, when the pore radius is not more than the upper limit value, this advantageously leads to an increase in the mechanical strength and a decrease in the pressure loss during passing of liquid.

2) The pore volume is from 0.1 to 2.5 mL/g per unit mass of the porous particles. The pore volume is more preferably 0.2 mL/g or more, still more preferably 0.5 mL/g or more, and is more preferably 1.5 mL/g or less, still more preferably

1.2 mL/g or less. When the pore volume satisfies the ranges above, this advantageously raises the prospect of ensuring adsorption sites for high molecules such as human insulin or protein and in turn, increasing the throughput. In addition, when the pore volume is not more than the upper limit value, this advantageously leads to a decrease in the void ratio within the particles and an increase in the adsorption amount per column volume.

3) The specific surface area is from 10 to 1,000 $m^2/g$ per unit mass of the porous particles. The specific surface area is more preferably 50 $m^2/g$ or more, still more preferably 100 $m^2/g$ or more, and is more preferably 500 $m^2/g$ or less, still more preferably 300 $m^2/g$ or less. When the specific surface area is not less than the lower limit value, the frequency of interaction between the insulin molecule and the separating agent surface increases, and this advantageously raises the prospect of enhancing the separation performance or increasing the throughput. In addition, when the specific surface area is not more than the upper limit value, this advantageously makes it possible to decrease an unnecessary interaction giving rise to deterioration of the separation performance.

4) The volume average particle diameter of the porous particles is from 1 to 50 μm. The volume average particle diameter is more preferably from 4 to 30 μm, still more preferably from 10 to 20 μm. When the volume average particle diameter is not more than the upper limit value and is smaller, a high theoretical plate number can be obtained. On the other hand, when the volume average particle diameter is not less than the lower limit value, this is advantageous in that the pressure loss decreases, the problem associated with equipment is relieved and industrial use is facilitated.

The volume average particle diameter, pore radius, pore volume and specific surface area of the separating agent for the purification of human insulin according to the second embodiment of the present invention are preferably within the same ranges as the characteristics of the above-described porous particles. Fundamentally, the particle diameter or pore properties of the separating agent for the purification of human insulin according to the second embodiment of the present invention reflect the characteristics of the porous particles used.

Incidentally, the volume average particle diameter is measured by the Coulter counter method. The sample is prepared as a dispersion with a predetermined aqueous sodium chloride solution, and an aperture for allowing the measured particle diameter to fall in the range of 2 to 60% of the aperture diameter is used. The measurement is performed by means of the Coulter counter using a suspension prepared by dispersing the sample in an aqueous 7 mass% sodium chloride solution such that the numerical value of the densitometer becomes from 5 to 10%. The average diameter of volume statistics obtained by the measurement is defined as the volume average particle diameter.

The separating agent for the purification of human insulin according to the second embodiment of the present invention having the above-described volume average particle diameter is obtained, for example, by a known classification method. As the classification method, for example, separation by a sieve, water separation using water flow, air separation using air flow, sedimentation classification using the difference in sedimentation rate, and centrifugal separation using the centrifugal force can be utilized. In addition, the separating agent for the purification of human insulin according to the second embodiment of the present invention having the above-described uniformity coefficient can also be obtained by the uniform particle size production technique described in Japanese Patent Application No. 63-116916 (JP-A-1-289802).

[Hydrophobic Functional Group]

The separating agent for the purification of human insulin according to the second embodiment of the present invention preferably includes porous particles, a hydrophobic functional group bonded to the porous particles, and an ion exchange group bonded to the porous particles, as described above.

It is preferred that the hydrophobic functional group is directly bonded by a reaction with a reactive group present on the surface of the porous particle.

The hydrophobic functional group is not particularly limited but includes, for example, a saturated alkyl group such as n-butyl group, tert-butyl group, n-octyl group and n-octadecyl group; an unsaturated alkyl group such as propenyl group, butenyl group, butadienyl group and hexenyl group; and an aryl group such as benzyl group, phenyl group and naphthyl group. The hydrophobic functional group is preferably at least one of a saturated alkyl group, an unsaturated alkyl group, and an aryl group, more preferably a saturated alkyl group having from 4 to 30 carbon atoms, still more preferably a saturated alkyl group having from 8 to 18 carbon atoms. When the chain length of the alkyl group is long, sufficient hydrophobic interaction can be exerted, and when the chain length is shorter than the upper limit value, the separation performance is advantageously enhanced without inhibiting diffusion of the objective substance into the particles.

The amount of the introduced hydrophobic functional group is, per unit mass of the separating agent in dry state, preferably from 0.01 g/g or more, more preferably 0.03 g/g or more, still more preferably 0.05 g/g or more and is preferably 0.5 g/g or less, more preferably 0.25 g/g or less, still more preferably 0.2 g/g or less.

The dry state as used in the present description is a state where water is evaporated under reduced pressure

at 50°C until the mass of the separating agent does not change.

When the amount of the introduced hydrophobic functional group is not more than the upper limit value, this is advantageous in that the hydrophobic interaction can be prevented from being exerted more than is necessary and inhibiting hydrophilic interaction to cause reduction in the separation performance, and when the amount is not less than the lower limit value, this is advantageous in that the hydrophobic interaction is sufficiently exerted and high separation performance is obtained.

[Ion Exchange Group]

The separating agent for the purification of human insulin according to the second embodiment of the present invention preferably includes porous particles, a hydrophobic functional group bonded to the porous particles, and an ion exchange group bonded to the porous particles, as described above.

It is preferred that the ion exchange group is directly bonded by a reaction with a reactive group present on the surface of the porous particles.

The ion exchange group is not particularly limited but is selected from, for example, an anion exchange group such as quaternary ammonium group, tertiary amino group, secondary amino group and primary amino group, and a cation exchange group such as sulfo group and carboxyl group. The ion exchange group is preferably an anion exchange group, more preferably a quaternary ammonium group, a tertiary amino group or a long-chain alkylamino group, still more preferably a long-chain alkylamino group.

An anion exchange group having a long-chain alkyl group is preferably introduced, because the steric freedom of the ion exchange group is increased to bring out stronger interaction with a target to be separated and the separation performance is enhanced. In the case of performing the purification of human insulin by using the separating agent for the purification of human insulin according to the second embodiment of the present invention, it is particularly preferable to use a long-chain alkylamino group as the ion exchange group.

The amount of the introduced ion exchange group is, per unit mass of the separating agent in dry state, preferably from 0.01 meq/g or more, more preferably 0.05 meq/g or more, still more preferably 0.1 meq/g or more and is preferably 2.0 meq/g or less, more preferably 1.0 meq/g or less, still more preferably 0.8 meq/g or less. When the amount of the introduced ion exchange group is not more than the upper limit value, this is advantageous in that the ionic interaction can be prevented from becoming stronger than is necessary and inhibiting the hydrophobic interaction to cause reduction in the separation performance, and when the amount is not less than the lower limit value, this is advantageous in that the ionic interaction is sufficiently exerted and high separation performance is obtained.

[Production Method of Separating Agent for Purification of Human Insulin]

The production method of the separating agent for the purification of human insulin according to the second embodiment of the present invention preferably includes a step of introducing a hydrophobic functional group and a step of introducing an ion exchange group, directly into the surface of the porous particles.

In the case of organic polymeric particles in which only a side chain having both a hydrophobic moiety and an ion exchanging moiety in one molecule is bonded as in conventional techniques, the hydrophobic interaction and the hydrophilic interaction may not be fully exerted, leading to insufficient separation performance.

The preferred embodiment using the separating agent for the purification of human insulin according to the second embodiment of the present invention has technical significance in terms of solving the problems above.

The step of introducing a hydrophobic functional group into the surface of the porous particles and the step of introducing an ion exchange group into the surface of the porous particles may be performed in any order or may be performed simultaneously, but a method of introducing a hydrophobic functional group by chemical bonding and subsequently introducing an ion exchange group by chemical bonding is preferred.

(Step of Introducing Hydrophobic Functional Group)

With respect to the introduction of a hydrophobic functional group, the hydrophobic function group is introduced by reacting, by a known method, a hydrophobic compound with a reactive group on the porous particle surface to achieve chemical bonding. The reactive group on the porous particle surface includes, as described above, a vinyl group, a chloromethyl group, an epoxy group, etc., and the hydrophobic compound is sufficient if it has a functional group capable of reacting with the reactive group to form a chemical bond. The hydrophobic compound is preferably, for example, a compound having a functional group such as halogen group (e.g., chlor group), amino group and thiol group, and includes, for example, a saturated aliphatic alkyl compound such as alkylthiol, alkylamine and alkyl halide. In particular, an alkyl group is preferably introduced by reacting an alkylthiol with a vinyl group and/or an epoxy group on the porous particles.

In introducing a hydrophobic functional group, it is particularly preferable to bring the porous particles into

contact with the hydrophobic compound in an appropriate solvent and introduce the hydrophobic functional group in the presence of a catalyst. The solvent is preferably, for example, a solvent that swells the porous particles and dissolves the hydrophobic compound, particularly, a saturated aliphatic alkyl compound, and the solvent is not particularly limited as long as it can disperse porous particles. In the case of using an alkylthiol as the hydrophobic compound, an alkali such as sodium hydroxide or lithium hydroxide is preferably used as the catalyst, and the reaction is preferably performed in a nitrogen atmosphere. Because, such a method can be easily conducted and moreover, provides good production efficiency of the separating agent for the purification of human insulin according to the second embodiment of the present invention.

(Step of Introducing ion Exchange Group)

With respect to the introduction of an ion exchange group, the ion exchange group is introduced by reacting, by a known method, an ionic compound with a reactive group on the porous particle surface to achieve chemical bonding. The reactive group on the porous particle surface includes, as described above, a vinyl group, a chloromethyl group, an epoxy group, etc., and the ionic compound is sufficient if it reacts with the reactive group to form a chemical bond and thereby forms an ion exchange group.

The ionic compound includes, for example, tertiary amines such as trimethylamine, triethylamine and diethylethanolamine; secondary amines such as dimethylamine and diethylamine; and primary amines such as 1,2-diaminoethane and 1,6-diaminohexane. In particular, a long-chain alkylamino group or a quaternary ammonium group is preferably introduced by using diaminoalkane, trimethylamine, etc. as the ionic compound and reacting it with an epoxy group on the porous particle surface.

In introducing an ion exchange group, it is particularly preferable to bring the porous particles into contact with the ionic compound in an appropriate medium and introduce the ionic exchange group by heating the medium. Because, such a method can be easily conducted and moreover, provides good production efficiency of the separating agent for the purification of human insulin according to the second embodiment of the present invention.

[Characteristic Features of Separating Agent for Purification of Human Insulin]

The separating agent for the purification of human insulin according to the second embodiment of the present invention is characterized in that at the time of isolating human insulin by use of the separating agent under the following liquid chromatography separation conditions from a solution containing human insulin represented by the following formula (1) and A21 desamidoinsulin represented by the following formula (3), the recovery rate of human insulin is 97% or more when recovered in 99.9% purity.

In view of purification efficiency, the recovery rate of human insulin is preferably higher and is preferably 98% or more, more preferably 99% or more.

(Liquid Chromatography Separation Conditions)

Column size: 150 mm×4.6 mm inner diameter
Eluent: water/pH adjusting agent/water-soluble organic solvent
Flow velocity: 1 mL/min
Column temperature: 25°C
Measurement wavelength: UV 280 nm
Sample: a 100 mg/mL solution prepared from an insulin mixture containing 96 mass% of human insulin represented by the following formula (1) and 4 mass% of A21 desamidoinsulin represented by the following formula (3)
Sample injection volume: 400 μL

[Chem. 6]

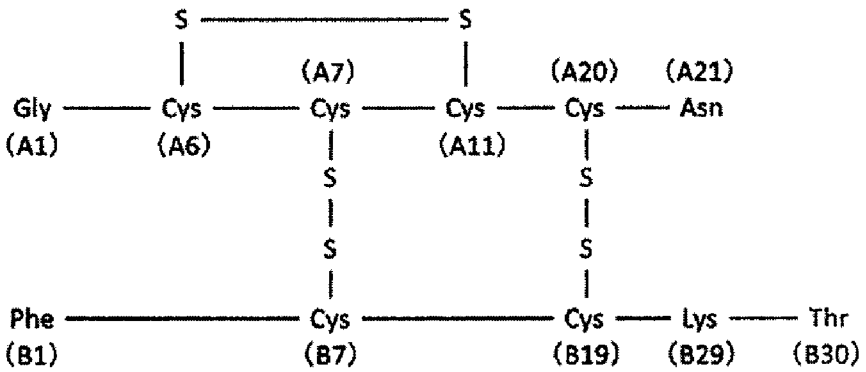

...(1)

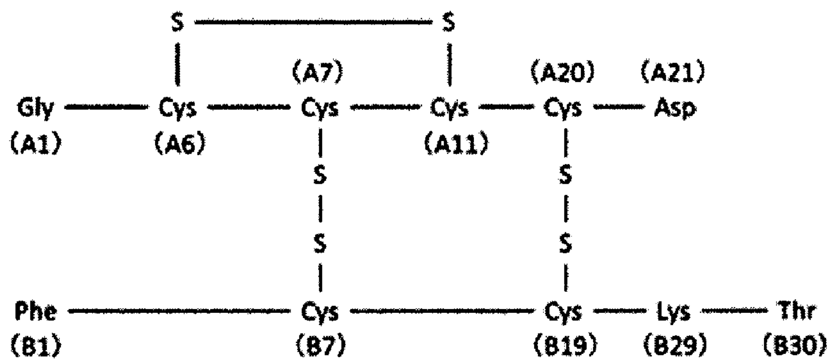

...(3)

The value of the recovery rate above can be appropriately adjusted by the volume average particle diameter, pore properties, amount of surface functional group and combination of functional group species in the separating agent used, and the organic solvent concentration and pH of the mobile phase in liquid chromatography.

The eluent for liquid chromatography may be sufficient if it fully dissolves human insulin, and the eluent contains substances generally used in liquid chromatography, such as water, pH adjusting agent and water-soluble organic solvent. The eluent is preferably similar in composition and pH to the solvent contained in the insulin solution 2 but is not necessarily limited thereto.

As the water-soluble organic solvent, for example, alcohols, acetonitrile, etc. are used and for obtaining high separation property and reducing the pressure during passing of liquid, acetonitrile is preferably used.

The water-soluble organic solvent concentration in the eluent may be appropriately changed in each step of liquid chromatography but is preferably from 5 to 60 vol%, more preferably 10 vol% or more, still more preferably 20 vol% or more, and is more preferably 50 vol% or less. When the water-soluble organic solvent concentration is 5 vol% or more, elution of insulin is facilitated, and when the concentration is 60 vol% or less, precipitation of salts added to the eluent can be suppressed.

As the eluent for liquid chromatography used in the second embodiment of the present invention, an eluent containing from 10 to 50 vol% of acetonitrile is particularly preferred.

In addition, the content of water in the eluent is preferably from 40 to 95 vol%, more preferably from 50 to 90 vol%, still more preferably from 60 to 80 vol%, in view of solubility of insulin, pH adjusting agent, etc.

Furthermore, the content of the pH adjusting agent in the eluent is preferably 0.01 mol/L or more per volume of the eluent so as to obtain sufficient pH adjusting ability and is preferably 1 mol/L or less so as not to inhibit interaction between the separating agent and insulin in chromatography.

As the pH adjusting agent, an acid, a base, and salts having a pH buffering ability are appropriately used. The pH adjusting agent includes, for example, an inorganic acid such as hydrochloric acid and phosphoric acid; salts of these inorganic acids; an organic acid such as acetic acid, citric acid and trifluoroacetic acid; and salts of these organic acids.

An organic acid and an organic acid salt are preferred, and a mixture system of acetic acid and sodium acetate is more preferred.

The pH of the eluent is preferably 5.0 or less or 6.0 or more, more preferably 4.0 or less or 7.0 or more. When the pH of the eluent is 5.0 or less or 6.0 or more, sufficient insulin solubility is achieved, and a trouble such as precipitation during purification can be suppressed.

The pH of the eluent in the elution step is preferably set according to the type of the separating agent used in liquid chromatography, particularly, the type of the ion exchange group. For example, the eluent is preferably set to a pH higher than the isoelectric point (from 5.3 to 5.4) of the target insulin to be purified in the case of using a separating agent into which an acidic functional group is introduced as the ion exchange group, and set to a pH lower than the isoelectric point of the target insulin to be purified in the case of using a separation agent into which a basic functional group is introduced as the ion exchange group.

(Uniformity Coefficient)

The separating agent for the purification of human insulin according to the second embodiment of the present invention preferably has a sharp particle size distribution, and the uniformity coefficient represented by the following calculation method is usually 1.7 or less, preferably 1.5 or less, more preferably 1.3 or less. When the uniformity coefficient is not more than the upper limit value, at the time of packing porous crosslinked polymer particles into the column for reverse phase chromatography separation, the theoretical plate number of the column increases, and sufficient separation property is obtained.

[Uniformity Coefficient Calculation Method]

The uniformity coefficient can be determined from 25% diameter and 75% diameter of volume statics obtained by measurement, according to the following formula (I):

$$\text{Uniformity coefficient} = \{25\%\ \text{diameter}\ (\mu\text{m})\}/\{75\%\ \text{diameter}\ (\mu\text{m})\} \qquad \text{(I)}$$

(Other Physical Properties)

The separating agent for the purification of human insulin according to the second embodiment of the present invention must be porous particles, because a space for allowing the target human insulin to be separated to diffuse into the particles is required.

<Human Insulin Purification Method>

The purification method according to the second embodiment of the present invention is characterized by using the separating agent for the purification of human insulin according to the second embodiment of the present invention.

The purification method according to the second embodiment of the present invention is conducted by liquid chromatography using a separating agent-packed column and includes a step of preparing the above-described insulin solution 2, a step of preparing a separating agent-packed column, a loading step to the column, and a step of passing an eluent through the column to elute respective components. The purification method according to the second embodiment of the present invention is described below for each step.

[Preparation Step of Insulin Solution 2]

The insulin solution 2 used in the purification method according to the second embodiment of the present invention is the same as the insulin solution 2 described in

<Separating Agent for Purification of Human Insulin>.

In the purification method according to the second embodiment of the present invention, human insulin can be isolated by separating and removing mainly A21 desamidoinsulin from the insulin solution 2.

[Preparation Step of Separating Agent-Packed Column]

The purification method according to the second embodiment of the present invention is conducted by liquid chromatography using the separating agent for the purification of human insulin according to the second embodiment of the present invention.
Therefore, it is required to prepare the separating agent and pack the separating agent into the column.
In the purification method according to the second embodiment of the present invention, the separating agent is used in the state of being packed into the packing column for liquid chromatography, and the structure and size of the column are not particularly limited. The method for packing the separating agent into the column is also not particularly limited, but in order to form a uniform packed bed, wet packing of packing the separating agent into the column by charging it in a slurry state after dispersion in a packing solvent is preferred.

[Loading Step to Separating Agent-Packed Column]

In the purification method according to the second embodiment of the present invention, the insulin solution 2 is loaded to the column packed with the separating agent for the purification of human insulin according to the second embodiment of the present invention, and the purification is conducted by liquid chromatography.
In the step of loading the insulin solution 2 to the column, human insulin and A21 desamidoinsulin are retained within the column due to interaction of the separating agent with human insulin and A21 desamidoinsulin. In this step, an impurity that does not interact with the separating agent and is not retained on the column, exits the column and is thereby removed.
The insulin solution 2 loaded is preferably prepared to make human insulin and A21 desamidoinsulin be sufficiently dissolved and contains substances generally used in liquid chromatography, such as water, pH adjusting agent, organic solvent and salts. The solvent contained in the insulin solution 2 is preferably similar in composition to the above-described eluent but is not necessarily limited thereto.
As the organic solvent, for example, a water-soluble organic solvent such as alcohols and acetonitrile is used and for obtaining high separation property and reducing the pressure during passing of liquid, acetonitrile is preferably used.
The organic solvent concentration in the insulin solution 2 is preferably 50 vol% or less, more preferably 30 vol% or less, so as to prevent unnecessary elution of insulin in the loading step. In addition, as for the pH of the insulin solution 2, in order to prevent insulin precipitation in the loading step and pH variation within the column in the subsequent elution step, the pH difference from the above-described eluent is preferably 1 or less, more preferably 0.5 or less.
The total concentration of human insulin concentration and A21 desamidoinsulin concentration in the insulin solution 2 may be appropriately selected depending on the purpose of purification but in view of insulin solubility and separation efficiency, the total concentration is preferably 1,000 mg/mL or less, more preferably 500 mg/mL or less, still more preferably 100 mg/mL or less, and in order to obtain sufficient purification efficiency, is preferably 0.1 mg/mL or more, more preferably 1 mg/mL or more. The total concentration of human insulin concentration and A21 desamidoinsulin concentration in the insulin solution 2 can be adjusted by the amount of water, organic solvent or other additives such as pH adjusting agent.
As the pH adjusting agent, an acid, a base, and salts having a pH buffering ability are appropriately used. The pH adjusting agent includes, for example, an inorganic acid such as hydrochloric acid and phosphoric acid; salts of these inorganic acids; an organic acid such as acetic acid, citric acid and trifluoroacetic acid; and salts of these organic acids. An organic acid and an organic acid salt are preferred, and a mixture system of acetic acid and sodium acetate is more preferred.
The pH of the insulin solution 2 after preparation is preferably 5.0 or less or 6.0 or more, more preferably 4.0 or less or 7.0 or more. When the pH of the insulin solution 2 is 5.0 or less or 6.0 or more, sufficient insulin solubility is achieved, and a trouble such as precipitation during purification can be suppressed.
The concentration of A21 desamidoinsulin contained in the insulin solution 2 is not particularly limited, but for the reason that when the amount of A21 desamidoinsulin is not more than a given amount relative to the amount of human insulin, the purification efficiency increases, the amount is preferably 50 mass% or less, more preferably 10 mass% or less, relative to the total amount of human insulin and A21 desamidoinsulin.
In addition, the amount of insulin loaded to the separating agent-packed column in one purification may be appropriately selected depending on the purpose of purification, but since when the amount loaded is not more than a given amount, the purification efficiency increases, the insulin is loaded in an amount of preferably 100 mg/mL or less, more preferably 50 mg/mL or less, relative to the volume of the separating agent packed into the column.
Before and after the loading of the insulin solution 2 to the column, if desired, the interior of the column is washed and equilibrated with a washing solution having the same or similar composition to the eluent used in the elution step. The organic solvent concentration in the washing solution is preferably not more than the concentration of the

organic solvent contained in the eluent so as to prevent unnecessary acceleration of the elution of insulin after loading. In addition, as for the pH of the washing solution, in order to prevent insulin precipitation after loading or pH variation within the column in the elution step, the pH difference from the above-described eluent is preferably 1 or less, more preferably 0.5 or less.

[Elution Step]

The purification method according to the second embodiment of the present invention includes a step of passing the above-described eluent through the insulin solution 2-loaded column to elute human insulin and perform elution of respective components. In particular, it is preferable to include a step of eluting human insulin with an eluent containing a water-soluble organic solvent, and in this case, gradient elution in which the water-soluble organic solvent concentration in the eluent is changed over time, and isocratic elution in which the water-soluble organic solvent concentration is constant, are used.

The separating agent for the purification of human insulin according to the second embodiment of the present invention can be suitably used for the purification of human insulin, because human insulin and A21 desamidoinsulin can be effectively separated.

The purification above can be performed by packing the separating agent for the purification of human insulin according to the second embodiment of the present invention into a column, and passing a human insulin solution containing A21 desamidoinsulin as an impurity through the column.

EXAMPLES

The present invention is described more specifically below by referring to Examples, but the present invention is not limited to the contents described in the following Examples as long as its gist is observed.

[Examples According to First Embodiment]

Examples according to the first embodiment of the present invention are described.

[Evaluation Method]

The methods for evaluating the separating agents for the purification of human insulin obtained in the following Production Examples, Examples and Comparative Examples are as follows.

<Volume Average Particle Diameter>

The volume average particle diameter was measured by the Coulter counter method. The sample was prepared as a dispersion with a predetermined aqueous sodium chloride solution, and an aperture for allowing the measured particle diameter to fall in the range of 2 to 60% of the aperture diameter was used. The measurement was performed by means of the Coulter counter (manufactured by Beckman Coulter Inc.) using a suspension prepared by dispersing the sample in an aqueous 7 mass% sodium chloride solution such that the numerical value of the densitometer becomes from 5 to 10%. The number of particles measured was set to 30,000, and the average diameter of volume statistics obtained by the measurement was defined as the volume average particle diameter.

<Specific Surface Area>

The specific surface area was measured by the nitrogen gas adsorption method. The nitrogen gas adsorption method is a method of determining the monolayer adsorbed amount by using the BET equation from the change in pressure before and after adsorption, and the specific surface area can be calculated from a cross-sectional area of one molecule of nitrogen gas.

The porous particles subjected to a drying treatment were weighed and measured for the specific surface area by means of Flowsorb III2310 manufactured by Micromeritics Instrument Corp.

<Pore Volume, Pore Radius>

The pore volume and pore radius were measured by the nitrogen gas adsorption method. The nitrogen gas adsorption method is a method of determining the pore diameter and pore volume from the pressure and adsorbed amount when nitrogen gas molecules are condensed within the pore.

The porous particles subjected to a drying treatment were weighed and measured for the adsorption-desorption isotherm by means of ASAP 2420 manufactured by Micromeritics Instrument Corp. The integral pore volume distribution and Log micropore volume distribution were plotted using the obtained adsorption-desorption isotherm, and the pore volume and pore radius were determined.

<Amount of Ion Exchange Group>

The amount of the ion exchange group was measured by the titration method. In the case of a cation exchange support, the ion exchange group on the surface was regenerated by immersing a wet-state support sample in 2 N hydrochloric acid, and the sample was then thoroughly filtered and water-washed until the filtrate became neutral. The sample after water washing was dispersed in a 5 mass% saline solution and titrated with 0.1 N sodium hydroxide, and from the titer until reaching the neutralization point, the amount of the ion exchange group per unit mass of the wet-state support was calculated.

<Production Example 1-1>

(Synthesis of Porous Particles by Seed Polymerization)

0.8 g of sodium dodecylsulfate and 200 g of deionized water were added to 1 g of uniform-particle size polymethyl methacrylate seed particles having a particle diameter of 2.1 μm to prepare a seed-particle aqueous dispersion. A monomer phase dispersion prepared by dispersing a monomer phase in which 40 g of ethylene glycol dimethacrylate as a monomer component, 60 g of toluene as a pore-forming agent, and 0.8 g of 2,2'-azobisisobutyronitrile as a polymerization initiator were mixed, in 1.82 g of sodium dodecylsulfate and 460 g of deionized water was added to the seed-particle aqueous dispersion above and stirred at room temperature for 20 hours, and the monomer phase was thereby absorbed by the seed particle.

Subsequently, 172 g of an aqueous 5 mass% polyvinyl alcohol (Gosenol (registered trademark) GH-20, produced by Nippon Synthetic Chemical Industry Co., Ltd.) solution, 0.086 g of sodium nitrite, and 29 g of deionized water were added to the resulting dispersion, and polymerization was conducted at 70°C for 2 hours to obtain polymer particles. The polymer particles were isolated and after removing the seed particle-derived polymer by performing toluene extraction operation, dried to obtain the powder of Porous Particles 1-1.

The volume average particle diameter of the obtained Porous Particles 1-1 was 10.0 μm. In addition, when the particles were measured by the nitrogen gas adsorption method, the pore volume was 1.02 mL/g, the specific surface area was 445 $m^2/g$, and the pore radius was 228 Å.

<Production Example 1-2>

Synthesis of Porous Particles 1-2 was performed under the same conditions as in Production Example 1-1 except that 28.6 g of ethylene glycol dimethacrylate, 71.4 g of toluene and 0.57 g of 2,2'-azobisisobutyronitrile were used.

The volume average particle diameter of the obtained Porous Particles 1-2 was 9.9 μm. In addition, when the particles were measured by the nitrogen gas adsorption method, the pore volume was 1.10 mL/g, the specific surface area was 501 $m^2/g$, and the pore radius was 255 Å.

<Production Example 1-3>

Synthesis of Porous Particles 1-3 was performed under the same conditions as in Production Example 1-1 except that particles having a volume average particle diameter of 1.7 μm were used as the seed particle.

The volume average particle diameter of the obtained Porous Particles 1-3 was 8.0 μm. In addition, when the particles were measured by the nitrogen gas adsorption method, the pore volume was 0.89 mL/g, the specific surface area was 420 $m^2/g$, and the pore radius was 197 Å.

<Production Example 1-4>

Synthesis of Porous Particles 1-4 was performed under the same conditions as in Production Example 1-3 except that in the seed polymerization stage, 2,2'-azobis(methyl isobutyrate) was used as the polymerization initiator.

The volume average particle diameter of the obtained Porous Particles 1-4 was 8.0 μm. In addition, when the particles were measured by the nitrogen gas adsorption method, the pore volume was 0.88 mL/g, the specific surface area was 434 $m^2/g$, and the pore radius was 176 Å.

<Production Example 1-5>

Synthesis of Porous Particles 1-5 was performed under the same conditions as in Production Example 1-3 except that in the seed polymerization stage, 28 g of ethylene glycol dimethacrylate and 12 g of glycerol dimethacrylate were used as the monomer component.

The volume average particle diameter of the obtained Porous Particles 1-5 was 8.0 μm. In addition, when the particles were measured by the nitrogen gas adsorption method, the pore volume was 0.43 mL/g, the specific surface area was 271 $m^2$/g, and the pore radius was 227 Å.

<Production Example 1-6>

Synthesis of Porous Particles 1-6 was performed under the same conditions as in Production Example 1-4 except that in the seed polymerization stage, 33.3 g of ethylene glycol dimethacrylate as the monomer component, 66.7 g of toluene as the pore-forming agent, and 0.67 g of 2,2'-azobis(methyl isobutyrate) as the polymerization initiator were used.

The volume average particle diameter of the obtained Porous Particles 1-6 was 8.0 μm. In addition, when the particles were measured by the nitrogen gas adsorption method, the pore volume was 0.98 mL/g, the specific surface area was 481 $m^2$/g, and the pore radius was 229 Å.

<Production Example 1-7>

Synthesis of Porous Particles 1-7 was performed under the same conditions as in Production Example 1-3 except that in the seed polymerization stage, 32 g of ethylene glycol dimethacrylate and 8 g of glycerol dimethacrylate were used as the monomer component.

The volume average particle diameter of the obtained Porous Particles 1-7 was 8.0 μm. In addition, when the particles were measured by the nitrogen gas adsorption method, the pore volume was 0.70 mL/g, the specific surface area was 269 $m^2$/g, and the pore radius was 200 Å.

<Production Example 1-8>

Synthesis of Porous Particles 1-8 was performed under the same conditions as in Production Example 1-3 except that in the seed polymerization stage, 28 g of ethylene glycol dimethacrylate and 12 g of glycerol dimethacrylate were used as the monomer component.

The volume average particle diameter of the obtained Porous Particles 1-8 was 8.0 μm. In addition, when the particles were measured by the nitrogen gas adsorption method, the pore volume was 0.64 mL/g, the specific surface area was 189 $m^2$/g, and the pore radius was 173 Å.

<Production Example 1-9>

Synthesis of Porous Particles 1-9 was performed under the same conditions as in Production Example 1-4 except that in the seed polymerization stage, 20 g of ethylene glycol dimethacrylate and 20 g of glycerol dimethacrylate were used as the monomer component.

The volume average particle diameter of the obtained Porous Particles 1-9 was 8.0 μm. In addition, when the particles were measured by the nitrogen gas adsorption method, the pore volume was 0.42 mL/g, the specific surface area was 105 $m^2$/g, and the pore radius was 252 Å.

<Example 1-1>

In a reactor equipped with a stirrer, a condenser, a thermometer and a nitrogen gas introducing tube, 3 g of Porous Particles 1-1 synthesized in Production Example 1-1, 50 g of demineralized water, 4.46 g of sodium mercaptopropanesulfonate, and 0.104 g of 2,2'-azobis[N-(2-carboxyethyl)-2-methylpropionamidine] n-hydrate (VA-057, produced by Wako Pure Chemical Industries, Ltd.) were placed and mixed, and the reaction was performed for 5 hours by heating the mixture at 70°C in a nitrogen atmosphere. After cooling the reaction solution, the particles were washed with demineralized water and filtered to obtain a wet-state ion exchange support (separating agent) into which a sulfonic acid group is introduced as the ion exchange group. With respect to the obtained wet-state ion exchange support (separating agent), the amount of the ion exchange group was measured by the titration method and found to be 0.095 meq/g.

The obtained ion exchange support (separating agent) was packed into the column, and human insulin was isolated from a human insulin/desB30 insulin mixture by liquid chromatography using the following conditions.

(Human Insulin Isolation Conditions)

Evaluation apparatus: HPLC system manufactured by Shimadzu Corporation
Column size: 150 mm×4.6 mm inner diameter
Eluent A1: acetic acid/water = 1/100 (volume ratio)
Eluent B1: acetic acid/2 M NaCl = 1/100 (volume ratio)
Eluent C1: acetic acid/acetonitrile = 1/100 (volume ratio)
Column equilibration conditions: Eluent B1: 0 vol%, Eluent C1: 35 vol%
Elution conditions:

Eluent B1: 0 vol% → 50 vol%/40 min, kept at 50 vol% for 5 minutes
Eluent C1: 35 vol% (constant)

Flow velocity: 1 mL/min
Column temperature: 25°C
Measurement wavelength: UV 280 nm
Sample: a 100 mg/mL solution prepared from an insulin mixture solution containing 96 mass% of human insulin represented by formula (1) and 4 mass% of desB30 insulin represented by formula (2)
Sample injection volume: 100 μL

The chromatography procedure is described below.

(Preparation of Insulin Solution)

An insulin solution was prepared to contain 96 mg/mL of human insulin and 4 mg/mL of desB30 insulin in an aqueous 3% acetic acid solution.

(Column Equilibration)

The column packed with the separating agent was equilibrated under the above-described equilibration conditions.

(Insulin Elution)

100 μL of the insulin solution prepared above was loaded to the equilibrated column, and chromatography separation was performed under the above-described elusion conditions using an HPLC apparatus. The eluate was monitored at UV 280 nm, and the portion containing human insulin and/or desB30 insulin was collected in fractions for every 0.33 mL.

(Measurement of Purity and Recovery Rate of Insulin)

Each fraction collected was analyzed using reverse phase HPLC column, and the contents of human insulin and desB30 insulin in each fraction were calculated to determine the human insulin recovery rate when the eluate was collected to achieve a human insulin purity of 99.5% under the above-described elution conditions.
The "human insulin purity" is a value calculated according to the following calculation formula in the eluate collected by the liquid chromatography.

$$\text{Purity (\%)} = \{\text{mass (g) of human insulin/mass (g) of human insulin + mass (g) of desB30 insulin)}\}\times 100$$

The "human insulin recovery rate" is a value calculated according to the following calculation formula in the loaded sample and collected eluate in the liquid chromatography.

$$\text{Recovery rate (\%)} = (\text{mass (g) of human insulin in eluate/mass (g) of human insulin in loaded sample}))\times 100$$

The human insulin recovery rate at a purity of 99.5% calculated in the measurement above was 75%.

<Example 1-2>

A wet-state ion exchange support (separating agent) into which a sulfonic acid group is introduced as the ion exchange group was obtained in the same manner as in Example 1-1 except that the porous particles were changed to Porous Particles 1-3 synthesized in Production Example 1-3 and the amount of 2,2'-azobis[N-(2-carboxyethyl)-2-methylpropionamidine] n-hydrate was changed to 1.04 g. With respect to the obtained wet-state ion exchange support (separating agent), the amount of the ion exchange group was measured by the titration method and found to be 0.113 meq/g.

The obtained ion exchange support (separating agent) was packed into the column, and human insulin was isolated from a human insulin/desB30 insulin mixture by liquid chromatography using the same conditions as in Example 1-1. The calculated human insulin recovery rate at a purity of 99.5% was 77%.

<Example 1-3>

In a reactor equipped with a stirrer, a condenser, a thermometer and a nitrogen gas introducing tube, 3 g of Porous Particles 1-4 synthesized in Production Example 1-4, 50 g of demineralized water, 5.18 g of 2-acrylamido-2-methylpropanesulfonic acid, 2.08 g of 48 mass% sodium hydroxide, 0.18 g of sodium mercaptopropanesulfonate, and 0.104 g of 2,2'-azobis[N-(2-carboxyethyl)-2-methylpropionamidine] n-hydrate were placed and mixed, and the reaction was performed for 5 hours by heating the mixture at 80°C in a nitrogen atmosphere. After cooling the reaction solution, the particles were washed with demineralized water and filtered to obtain a wet-state ion exchange support (separating agent) into which a sulfonic acid group bonded to a water-soluble polymer is introduced as the ion exchange group. With respect to the obtained wet-state ion exchange support (separating agent), the amount of the ion exchange group was measured by the titration method and found to be 0.205 meq/g.

The obtained ion exchange support (separating agent) was packed into the column, and human insulin was isolated from a human insulin/desB30 insulin mixture by liquid chromatography using the same conditions as in Example 1-1. The calculated human insulin recovery rate at a purity of 99.5% was 79%.

<Example 1-4>

A wet-state ion exchange support (separating agent) into which a sulfonic acid group is introduced as the ion exchange group was obtained in the same manner as in Example 1-3 except that the porous particles were changed to Porous Particles 1-5 synthesized in Production Example 1-5. With respect to the obtained wet-state ion exchange support (separating agent), the amount of the ion exchange group was measured by the titration method and found to be 0.106 meq/g.

The obtained ion exchange support (separating agent) was packed into the column, and human insulin was isolated from a human insulin/desB30 insulin mixture by liquid chromatography using the same conditions as in Example 1-1. The calculated human insulin recovery rate at a purity of 99.5% was 86%.

<Example 1-5>

A wet-state ion exchange support (separating agent) into which a sulfonic acid group is introduced as the ion exchange group was obtained in the same manner as in Example 1-3 except that the porous particles were changed to Porous Particles 1-6 synthesized in Production Example 1-6. With respect to the obtained wet-state ion exchange support (separating agent), the amount of the ion exchange group was measured by the titration method and found to be 0.185 meq/g.

The obtained ion exchange support (separating agent) was packed into the column, and human insulin was isolated from a human insulin/desB30 insulin mixture by liquid chromatography using the same conditions as in Example 1-1. The calculated human insulin recovery rate at a purity of 99.5% was 86%.

<Example 1-6>

A wet-state ion exchange support intermediate into which a sulfonic acid group is introduced as the ion exchange

group was obtained in the same manner as in Example 1-3 except that the porous particles were changed to Porous Particles 1-7 synthesized in Production Example 1-7. With respect to the obtained wet-state ion exchange support intermediate, the amount of the ion exchange group was measured by the titration method and found to be 0.135 meq/g. The remaining epoxy group on the particle surface was ring-opened by treating the obtained ion exchange support intermediate in 10 mass% sulfuric acid at 60°C for 5 hours, as a result, a wet-state ion exchange support (separating agent) was obtained.

The obtained ion exchange support (separating agent) was packed into the column, and human insulin was isolated from a human insulin/desB30 insulin mixture by liquid chromatography using the same conditions as in Example 1-1 except that the proportion of Eluent C1 in the column equilibration conditions and elution conditions was changed to 25 vol%. The calculated human insulin recovery rate at a purity of 99.5% was 93%.

<Example 1-7>

The ion exchange support (separating agent) obtained in Example 1-6 was packed into the column, and human insulin was isolated from a human insulin/desB30 insulin mixture by liquid chromatography using the same conditions as in Example 1-1 except that the proportion of Eluent C1 in the column equilibration conditions and elution conditions was changed to 30 vol%. The calculated human insulin recovery rate at a purity of 99.5% was 92%.

<Example 1-8>

In a reactor equipped with a stirrer, a condenser, a thermometer and a nitrogen gas introducing tube, 6 g of Porous Particles 1-8 synthesized in Production Example 1-8, 100 g of demineralized water, 3.56 g of sodium mercaptopropanesulfonate, and 0.207 g of 2,2'-azobis[N-(2-carboxyethyl)-2-methylpropionamidine] n-hydrate were placed and mixed, and the reaction was performed for 5 hours by heating the mixture at 70°C in a nitrogen atmosphere. After cooling the reaction solution, the particles were washed with demineralized water and filtered to obtain a wet-state ion exchange support intermediate into which a sulfonic acid group is introduced as the ion exchange group.

With respect to the obtained wet-state ion exchange support intermediate, the amount of the ion exchange group was measured by the titration method and found to be 0.059 meq/g. The remaining epoxy group on the particle surface was ring-opened by treating the obtained ion exchange support intermediate in 10 mass% sulfuric acid at 60°C for 5 hours, as a result, a wet-state ion exchange support (separating agent) was obtained.

The obtained ion exchange support (separating agent) was packed into the column, and human insulin was isolated from a human insulin/desB30 insulin mixture by liquid chromatography using the same conditions as in Example 1-1 except that the proportion of Eluent C1 in the column equilibration conditions and elution conditions was changed to 25 vol%. The calculated human insulin recovery rate at a purity of 99.5% was 73%.

<Example 1-9>

A wet-state ion exchange support intermediate into which a sulfonic acid group is introduced as the ion exchange group was obtained in the same manner as in Example 1-6 except that the porous particles were changed to Porous Particles 1-8 synthesized in Production Example 1-8. With respect to the obtained wet-state ion exchange support intermediate, the amount of the ion exchange group was measured by the titration method and found to be 0.107 meq/g. The remaining epoxy group on the particle surface was ring-opened by treating the obtained ion exchange support intermediate in 10 mass% sulfuric acid at 60°C for 5 hours, as a result, a wet-state ion exchange support (separating agent) was obtained.

The obtained ion exchange support (separating agent) was packed into the column, and human insulin was isolated from a human insulin/desB30 insulin mixture by liquid chromatography using the same conditions as in Example 1-1 except that the proportion of Eluent C1 in the column equilibration conditions and elution conditions was changed to 25 vol%. The calculated human insulin recovery rate at a purity of 99.5% was 96%.

<Example 1-10>

The ion exchange support (separating agent) obtained in Example 1-9 was packed into the column, and human insulin was isolated from a human insulin/desB30 insulin mixture by liquid chromatography using the same conditions as in Example 1-1 except that the proportion of Eluent C1 in the column equilibration conditions and elution conditions was changed to 30 vol%. The calculated human insulin recovery rate at a purity of 99.5% was 96%.

<Example 1-11>

In a reactor equipped with a stirrer, a condenser, a thermometer and a nitrogen gas introducing tube, 3 g of Porous Particles 1-8 synthesized in Production Example 1-8, 50 g of demineralized water, 2.59 g of 2-acrylamido-2-methylpropanesulfonic acid, 1.44 g of hydroxyethylacrylamide, 1.04 g of 48 mass% sodium hydroxide, 0.18 g of sodium mercaptopropanesulfonate, and 0.104 g of 2,2'-azobis[N-(2-carboxyethyl)-2-methylpropionamidine] n-hydrate were placed and mixed, and the reaction was performed for 5 hours by heating the mixture at 80°C in a nitrogen atmosphere. After cooling the reaction solution, the particles were washed with demineralized water and filtered to obtain a wet-state ion exchange support intermediate into which a sulfonic acid group is introduced as the ion exchange group.

With respect to the obtained wet-state ion exchange support intermediate, the amount of the ion exchange group was measured by the titration method and found to be 0.054 meq/g. The remaining epoxy group on the particle surface was ring-opened by treating the obtained ion exchange support intermediate in 10 mass% sulfuric acid at 60°C for 5 hours, as a result, a wet-state ion exchange support (separating agent) was obtained.

The obtained ion exchange support (separating agent) was packed into the column, and human insulin was isolated from a human insulin/desB30 insulin mixture by liquid chromatography using the same conditions as in Example 1-1 except that the proportion of Eluent C1 in the column equilibration conditions and elution conditions was changed to 20 vol%. The calculated human insulin recovery rate at a purity of 99.5% was 84%.

<Example 1-12>

A wet-state ion exchange support intermediate into which a sulfonic acid group is introduced as the ion exchange group was obtained in the same manner as in Example 1-6 except that the porous particles were changed to Porous Particles 1-9 synthesized in Production Example 1-9. With respect to the obtained wet-state ion exchange support intermediate, the amount of the ion exchange group was measured by the titration method and found to be 0.047 meq/g. The remaining epoxy group on the particle surface was ring-opened by treating the obtained ion exchange support intermediate in 10 mass% sulfuric acid at 60°C for 5 hours, as a result, a wet-state ion exchange support (separating agent) was obtained.

The obtained ion exchange support (separating agent) was packed into the column, and human insulin was isolated from a human insulin/desB30 insulin mixture by liquid chromatography using the same conditions as in Example 1-1 except that the proportion of Eluent C1 in the column equilibration conditions and elution conditions was changed to 20 vol%. The calculated human insulin recovery rate at a purity of 99.5% was 87%.

<Comparative Example 1-1>

A wet-state ion exchange support (separating agent) into which a sulfonic acid group is introduced as the ion exchange group was obtained in the same manner as in Example 1-1 except that the porous particles were changed to Porous Particles 1-2 synthesized in Production Example 1-2 and the amount of sodium mercaptopropanesulfonate was changed to 0.45 g. With respect to the obtained wet-state ion exchange support (separating agent), the amount of the ion exchange group was measured by the titration method and found to be 0.043 meq/g.

The obtained ion exchange support (separating agent) was packed into the column, and human insulin was isolated from a human insulin/desB30 insulin mixture by liquid chromatography using the same conditions as in Example 1-1. The calculated human insulin recovery rate at a purity of 99.5% was 67%.

<Comparative Example 1-2>

A wet-state ion exchange support (separating agent) into which a sulfonic acid group is introduced as the ion exchange group was obtained in the same manner as in Example 1-2 except that the amount of sodium mercaptopropanesulfonate was changed to 0.18 g and the amount of 2,2'-azobis[N-(2-carboxyethyl)-2-methylpropionamidine] n-hydrate was changed to 0.01 g. With respect to the obtained wet-state ion exchange support (separating agent), the amount of the ion exchange group was measured by the titration method and found to be 0.024 meq/g.

The obtained ion exchange support (separating agent) was packed into the column, and human insulin was isolated from a human insulin/desB30 insulin mixture by liquid chromatography using the same conditions as in Example 1-1. The calculated human insulin recovery rate at a purity of 99.5% was 57%.

The results of these Examples and Comparative Examples are shown in Table 1.

[Table 1]

Table 1

| | Porous Particles | Volume Average Particle Diameter (μm) | Ion Exchange Group | | Eluent C1 (vol%) | Human Insulin Recovery Rate |
|---|---|---|---|---|---|---|
| | | | Functionalizing Agent | Amount Introduced (meq/g) | | |
| Example 1-1 | Production Example 1-1 | 10.0 | MPS-Na | 0.095 | 35 | 75% |
| Example 1-2 | Production Example 1-3 | 8.0 | MPS-Na | 0.113 | 35 | 77% |
| Example 1-3 | Production Example 1-4 | 8.0 | AMPS | 0.205 | 35 | 79% |
| Example 1-4 | Production Example 1-5 | 8.0 | AMPS | 0.106 | 35 | 86% |
| Example 1-5 | Production Example 1-6 | 8.0 | AMPS | 0.185 | 35 | 86% |
| Example 1-6 | Production Example 1-7 | 8.0 | AMPS | 0.135 | 25 | 93% |
| Example 1-7 | Production Example 1-7 | 8.0 | AMPS | 0.135 | 30 | 92% |
| Example 1-8 | Production Example 1-8 | 8.0 | MPS-Na | 0.059 | 25 | 73% |
| Example 1-9 | Production Example 1-8 | 8.0 | AMPS | 0.107 | 25 | 96% |
| Example 1-10 | Production Example 1-8 | 8.0 | AMPS | 0.107 | 30 | 96% |
| Example 1-11 | Production Example 1-8 | 8.0 | AMPS/HEAA | 0.054 | 20 | 84% |
| Example 1-12 | Production Example 1-9 | 8.0 | AMPS | 0.047 | 20 | 87% |
| Comparative Example 1-1 | Production Example 1-2 | 9.9 | MPS-Na | 0.043 | 35 | 67% |

(continued)

| | Porous Particles | Volume Average Particle Diameter (μm) | Ion Exchange Group | | Eluent C1 (vol%) | Human Insulin Recovery Rate |
|---|---|---|---|---|---|---|
| | | | Functionalizing Agent | Amount Introduced (meq/g) | | |
| Comparative Example 1-2 | Production Example 1-3 | 8.0 | MPS-Na | 0.024 | 35 | 57% |
| MPS-Na: sodium mercaptopropanesulfonate<br>AMPS: 2-acrylamido-2-methylpropanesulfonic acid<br>HEAA: hydroxyethylacrylamide | | | | | | |

[Examples According to Second Embodiment]

Examples according to the second embodiment of the present invention are described.

[Evaluation Method]

The methods for evaluating the separating agents for the purification of human insulin obtained in the following Production Examples, Examples and Comparative Examples are as follows.

<Volume Average Particle Diameter>

The volume average particle diameter was measured by the Coulter counter method. The sample was prepared as a dispersion with a predetermined aqueous sodium chloride solution, and an aperture for allowing the measured particle diameter to fall in the range of 2 to 60% of the aperture diameter was used. The measurement was performed by means of the Coulter counter (manufactured by Beckman Coulter Inc.) using a suspension prepared by dispersing the sample in an aqueous 7 mass% sodium chloride solution such that the numerical value of the densitometer becomes from 5 to 10%. The number of particles measured was set to 30,000, and the average diameter of volume statistics obtained by the measurement was defined as the volume average particle diameter.

<Specific Surface Area>

The specific surface area was measured by the nitrogen gas adsorption method. The nitrogen gas adsorption method is a method of determining the monolayer adsorbed amount by using the BET equation from the change in pressure before and after adsorption, and the specific surface area can be calculated from a cross-sectional area of one molecule of nitrogen gas.
The porous particles subjected to a drying treatment were weighed and measured for the specific surface area by means of Flowsorb III2310 manufactured by Micromeritics Instrument Corp.

<Pore Volume, Pore Radius>

The pore volume and pore radius were measured by the nitrogen gas adsorption method. The nitrogen gas adsorption method is a method of determining the pore diameter and pore volume from the pressure and adsorbed amount when nitrogen gas molecules are condensed within the pore.
The porous particles subjected to a drying treatment were weighed and measured for the adsorption-desorption isotherm by means of ASAP 2420 manufactured by Micromeritics Instrument Corp. The integral pore volume distribution and Log micropore volume distribution were plotted using the obtained adsorption-desorption isotherm, and the pore volume and pore radius were determined.

<Amount of Ion Exchange Group>

The amount of the ion exchange group was measured by the titration method. In the case of an anion exchange support, the ion exchange group on the surface was regenerated by immersing a wet-state support sample in 2 N sodium hydroxide, and the sample was then thoroughly filtered and water-washed until the filtrate became neutral. The sample

after water washing was dispersed in a 5 mass% saline solution and titrated with 0.1 N hydrochloric acid, and the titer until reaching the neutralization point was determined. Thereafter, from the mass when the sample having further been water-washed and filtered was dried, the amount of the ion exchange group per unit mass of the separating agent was calculated.

<Production Example 2-1>

(Synthesis of Porous Particles by Seed Polymerization)

0.8 g of sodium dodecylsulfate and 200 g of deionized water were added to 1 g of uniform-particle size polymethyl methacrylate seed particles having a particle diameter of 1.7 μm to prepare a seed-particle aqueous dispersion. A monomer phase dispersion prepared by dispersing a monomer phase in which 40 g of ethylene glycol dimethacrylate and 10 g of glycidyl methacrylate as monomer components, 50 g of toluene as a pore-forming agent, and 1 g of 2,2'-azobisisobutyronitrile as a polymerization agent were mixed, in 1.82 g of sodium dodecylsulfate and 460 g of deionized water was added to the seed-particle aqueous dispersion above and stirred at room temperature for 20 hours, and the monomer phase was thereby absorbed by the seed particle.

Subsequently, 172 g of an aqueous 5 mass% polyvinyl alcohol (Gosenol (registered trademark) GH-20, produced by Nippon Synthetic Chemical Industry Co., Ltd.) solution, 0.086 g of sodium nitrite, and 29 g of deionized water were added to the resulting dispersion, and polymerization was conducted at 70°C for 2 hours to obtain polymer particles. The polymer particles were isolated and after removing the seed particle-derived polymer by performing toluene extraction operation, dried to obtain the powder of Porous Particles 2-1.

The volume average particle diameter of the obtained Porous Particles 2-1 was 8.0 μm. In addition, when the particles were measured by the nitrogen gas adsorption method, the pore volume was 0.63 mL/g, the specific surface area was 250 $m^2/g$, and the pore radius was 173 Å.

<Production Example 2-2>

Synthesis of Porous Particles 2-2 was performed under the same conditions as in Production Example 2-1 except that 28 g of ethylene glycol dimethacrylate, 12 g of glycidyl methacrylate, 60 g of toluene and 0.8 g of 2,2'-azobisisobutyronitrile were used.

The volume average particle diameter of the obtained Porous Particles 2-2 was 8.0 μm. In addition, when the particles were measured by the nitrogen gas adsorption method, the pore volume was 0.61 mL/g, the specific surface area was 161 $m^2/g$, and the pore radius was 193 Å.

<Production Example 2-3>

Synthesis of Porous Particles 2-3 was performed under the same conditions as in Production Example 2-1 except that 32 g of ethylene glycol dimethacrylate, 8 g of glycidyl methacrylate, 60 g of toluene and 0.8 g of 2,2'-azobisisobutyronitrile were used.

The volume average particle diameter of the obtained Porous Particles 2-3 was 8.0 μm. In addition, when the particles were measured by the nitrogen gas adsorption method, the pore volume was 0.75 mL/g, the specific surface area was 268 $m^2/g$, and the pore radius was 229 Å.

<Production Example 2-4>

Synthesis of Porous Particles 2-4 was performed under the same conditions as in Production Example 2-1 except that the particle diameter of the seed particles was changed to 2.1 μm and 40 g of ethylene glycol dimethacrylate as a monomer component, 60 g of toluene and 0.8 g of 2,2'-azobisisobutyronitrile were used.

The volume average particle diameter of the obtained Porous Particles 2-4 was 10.0 μm. In addition, when the particles were measured by the nitrogen gas adsorption method, the pore volume was 0.90 mL/g, the specific surface area was 450 $m^2/g$, and the pore radius was 196 Å.

<Example 2-1>

(Introduction of Hydrophobic Functional Group)

5.00 g of Porous Particles 2-1 synthesized in Production Example 2-1 was dispersed in 50 mL of tetrahydrofuran, and 2.26 g of octadecylmercaptan was added, followed by stirring at 10°C for 30 minutes. In a nitrogen atmosphere, 5

mL of an aqueous lithium hydroxide solution (19.44 g/L) was added as a catalyst, and the reaction was performed while gently stirring the mixture at 25°C for 7 hours. After the completion of reaction, the obtained particles were washed with water, methanol and toluene and further washed with methanol and water to obtain hydrophobized porous particles. The rate of increase in mass of the porous particles due to introduction of a hydrophobic functional group was 13.6% in a dry state.

(Introduction of Ion Exchange Group)

10.0 g of the hydrophobized porous particles above were dispersed in 30 mL of water and heated at 50°C, 12.7 mL of an aqueous trimethylamine solution (58.6 g/L) was added dropwise, and the reaction was performed for 5 hours while gently stirring the mixture. After the completion of reaction, the interior of the system was neutralized with 2 mol/L hydrochloric acid, and the particles were separated by filtration and washed with water until the filtrate became neutral, to obtain a separating agent.

The ion exchange capacity per dry mass of the obtained separating agent was 0.200 meq/g.

(Insulin Separation Test)

The separating agent obtained above was packed into the column, and human insulin was isolated from a human insulin/A21 desamidoinsulin mixture by liquid chromatography using the following conditions.

(Human Insulin Isolation Conditions)

Evaluation apparatus: HPLC system manufactured by Shimadzu Corporation
Column size: 150 mm×4.6 mm inner diameter
Eluent A2: 2.5 M sodium acetate buffer solution (pH 4.5)/water/acetonitrile = 10/80/10 (volume ratio)
Eluent B2: 2.5 M sodium acetate buffer solution (pH 4.5)/water/acetonitrile = 10/40/50 (volume ratio)
Column equilibration conditions: Eluent B2: 30 vol%
Elution conditions:
Eluent B2: 30 vol% → 65 vol%/35 min, kept at 65 vol% for 2 minutes
Column temperature: 25°C
Flow velocity: 1 mL/min
Measurement wavelength: UV 280 nm
Sample: a 100 mg/mL solution prepared from an insulin mixture solution containing 96 mass% of human insulin and 4 mass% of A21 desamidoinsulin
Sample injection volume: 400 μL

The chromatography procedure is described below.

(Preparation of Insulin Solution)

An insulin solution was prepared to contain 96 mg/mL of human insulin and 4 mg/mL of A21 desamidoinsulin in an aqueous 3% acetic acid solution.

(Column Equilibration)

The column packed with the separating agent was equilibrated under the above-described equilibration conditions.

(Insulin Elution)

100 μL of the insulin solution prepared above was loaded to the equilibrated column, and chromatography separation was performed under the above-described elusion conditions using an HPLC apparatus. The eluate was monitored at UV 280 nm, and the portion containing human insulin and/or A21 desamidoinsulin was collected in fractions for every 0.6 mL.

(Measurement of Purity and Recovery Rate of Insulin)

Each fraction collected was analyzed using reverse phase HPLC column, and the contents of human insulin and A21 desamidoinsulin in each fraction were calculated to determine the human insulin recovery rate when the eluate was collected to achieve a human insulin purity of 99.9% under the above-described elution conditions.

The "human insulin purity" is a value calculated according to the following calculation formula in the eluate collected by the liquid chromatography.

$$\text{Purity (\%)} = \{\text{mass (g) of human insulin/mass (g) of human insulin + mass (g) of A21 desamidoinsulin)}\}\times 100$$

The "insulin recovery rate" is a value calculated according to the following calculation formula in the loaded sample and collected eluate in the liquid chromatography.

$$\text{Recovery rate (\%)} = (\text{mass (g) of human insulin in eluate/mass (g) of human insulin in loaded sample)}\}\times 100$$

The human insulin recovery rate at a purity of 99.9% calculated by analyzing the eluate in the measurement above by reverse phase chromatography was 98%.

<Example 2-2>

(Introduction of Hydrophobic Functional Group)

5.00 g of Porous Particles 2-2 synthesized in Production Example 2-2 was dispersed in 60 mL of tetrahydrofuran, and 3.39 g of octadecylmercaptan was added, followed by stirring at 10°C for 30 minutes. In a nitrogen atmosphere, 5 mL of an aqueous lithium hydroxide solution (29.4 g/L) was added as a catalyst, and the reaction was performed while gently stirring the mixture at 25°C for 7 hours. After the completion of reaction, the obtained particles were washed with water, methanol and toluene and further washed with methanol and water to obtain hydrophobized porous particles. The rate of increase in mass of the porous particles due to introduction of a hydrophobic functional group was 8.5% in a dry state.

(Introduction of Ion Exchange Group)

A separating agent was obtained by introducing an ion exchange group under the same conditions as in Example 2-1 except that the hydrophobized porous particles obtained above were used and 11.7 g of an aqueous dimethylamine solution (0.073 g/g) was used in place of the aqueous trimethylamine solution.

The ion exchange capacity per dry mass of the obtained separating agent was 0.171 meq/g.

(Insulin Separation Test)

The separating agent obtained above was packed into the column, and human insulin was isolated from a human insulin/A21 desamidoinsulin mixture by liquid chromatography using the same conditions as in Example 2-1. The calculated human insulin recovery rate at a purity of 99.9% was 99% or more.

<Example 2-3>

(Introduction of Hydrophobic Functional Group)

5.00 g of Porous Particles 2-2 synthesized in Production Example 2-2 was dispersed in 50 mL of diglyme, and 2.24 g of octadecylmercaptan was added, followed by stirring at 10°C for 30 minutes. In a nitrogen atmosphere, 5 mL of an aqueous lithium hydroxide solution (19.44 g/L) was added as a catalyst, and the reaction was performed while gently stirring the mixture at 25°C for 5 hours. After the completion of reaction, the obtained particles were washed with water, methanol and toluene and further washed with methanol and water to obtain hydrophobized porous particles.

The rate of increase in mass of the porous particles due to introduction of a hydrophobic functional group was 14.1% in a dry state.

(Introduction of Ion Exchange Group)

A separating agent was obtained by introducing an ion exchange group under the same conditions as in Example 2-1 except that the hydrophobized porous particles obtained above were used and 12.2 g of an aqueous 1,6-diaminohexane solution (0.18 g/g) was used in place of the aqueous trimethylamine solution.
The ion exchange capacity per dry mass of the obtained separating agent was 0.509 meq/g.

(Insulin Separation Test)

The separating agent obtained above was packed into the column, and human insulin was isolated from a human insulin/A21 desamidoinsulin mixture by liquid chromatography using the same conditions as in Example 2-1. The calculated human insulin recovery rate at a purity of 99.9% was 99% or more.

<Example 2-4>

(Introduction of Hydrophobic Functional Group)

Hydrophobized porous particles were obtained by introducing a hydrophobic functional group under the same conditions as in Example 2-1 except that Porous Particles 2-3 synthesized in Production Example 2-3 was used and 1.13 g of octylmercaptan was used in place of the octadecylmercaptan. The rate of increase in mass of the porous particles due to introduction of a hydrophobic functional group was 6.1% in a dry state.

(Introduction of Ion Exchange Group)

A separating agent was obtained by introducing an ion exchange group under the same conditions as in Example 2-1 except that the hydrophobized porous particles obtained above were used and 11.5 g of 1,6-diaminohexane (0.13 g/g) was used in place of the trimethylamine.
The ion exchange capacity per dry mass of the obtained separating agent was 0.312 meq/g.

(Insulin Separation Test)

The separating agent obtained above was packed into the column, and human insulin was isolated from a human insulin/A21 desamidoinsulin mixture by liquid chromatography using the same conditions as in Example 2-1. The calculated human insulin recovery rate at a purity of 99.9% was 99% or more.

<Comparative Example 2-1>

(Introduction of Ion Exchange Group)

A separating agent into which only an ion exchange group was introduced was obtained by introducing an ion exchange group under the same conditions as in Example 2-4 except that Porous Particles 2-3 synthesized in Production Example 2-3 was used in place of the hydrophobized porous particles obtained in Example 2-4.
The ion exchange capacity per dry mass of the obtained separating agent was 0.871 meq/g.

(Insulin Separation Test)

A separation test between human insulin and A21 desamidoinsulin was performed under the same conditions as in Example 2-1 by using a separating agent-packed column in which the separating agent obtained above was packed into a stainless steel-made column having an inner diameter of 4.6 mm and a length of 150 mm by a wet method, but human insulin and A21 desamidoinsulin were little retained, and the calculated human insulin recovery rate at a purity of 99.9% was less than 10%.

<Comparative Example 2-2>

(Introduction of Hydrophobic Functional Group)

Porous particles (separating agent) in which only a hydrophobic functional group was introduced by introducing the hydrophobic functional group under the same conditions as in Example 2-1 except that Porous Particles 2-4 synthesized in Production Example 2-4 was used in place of Porous Particles 2-1, were obtained. The rate of increase in mass of the porous particles due to introduction of a hydrophobic functional group was 12.3% in a dry state.

(Insulin Separation Test)

The separating agent obtained above was packed into the column, and human insulin was isolated from a human insulin/A21 desamidoinsulin mixture by liquid chromatography using the same conditions as in Example 2-1. The calculated human insulin recovery rate at a purity of 99.9% was 85%.

<Comparative Example 2-3>

(Insulin Separation Test)

With use of a commercially available column for reverse phase chromatography in which a silica separating agent is packed (Kromasil (registered trademark) 100-10-C8, produced by Akzo Nobel), human insulin was isolated from a human insulin/A21 desamidoinsulin mixture by liquid chromatography using the same conditions as in Example 2-1. The calculated human insulin recovery rate at a purity of 99.9% was 96%.

The results of these Examples and Comparative Examples are shown in Table 2.

[Table 2]

Table 2

| | Porous Particles | Volume Average Particle Diameter (μm) | Hydrophobic Functional Group | | | Ion Exchange Group | | Human Insulin Recovery Rate |
|---|---|---|---|---|---|---|---|---|
| | | | Type | Rate of Increase in Mass (%) | Amount Introduced (g/g) | Functionalizing Agent | Amount Introduced (meq/g) | |
| Example 2-1 | Production Example 2-1 | 8.0 | C18 | 13.6 | 0.120 | TMA | 0.200 | 98% |
| Example 2-2 | Production Example 2-2 | 8.0 | C18 | 8.5 | 0.078 | DMA | 0.171 | >99% |
| Example 2-3 | Production Example 2-2 | 8.0 | C18 | 14.1 | 0.124 | HMDA | 0.509 | >99% |
| Example 2-4 | Production Example 2-3 | 8.0 | C8 | 6.1 | 0.057 | HMDA | 0.312 | >99% |
| Comparative Example 2-1 | Production Example 2-3 | 8.0 | - | - | - | HMDA | 0.871 | <10% |
| Comparative Example 2-2 | Production Example 2-4 | 10.0 | C18 | 12.3 | 0.110 | - | - | 85% |
| Comparative Example 2-3 | - | 10.0 | C8 | - | - | - | - | 96% |

C18: octadecyl group
C8: octyl group
TMA: trimethylamine
DMA: dimethylamine
HMDA: 1,6-diaminohexane

While the invention has been described in detail and with reference to specific embodiments thereof, it will be apparent to one skilled in the art that various changes and modifications can be made therein without departing from the spirit and scope of the invention. This application is based on Japanese Patent Application (Patent Application No. 2017-023336) filed on February 10, 2017, Japanese Patent Application (Patent Application No. 2017-023337) filed on February 10, 2017, Japanese Patent Application (Patent Application No. 2017-061971) filed on March 27, 2017, and Japanese Patent Application (Patent Application No. 2017-122533) filed on June 22, 2017, the contents of which are incorporated herein by way of reference.

## Claims

1. A separating agent for purification of human insulin,
wherein at the time of isolating human insulin represented by the following formula (1) under the following liquid chromatography separation conditions by using the separating agent from a solution containing human insulin represented by the following formula (1) and desB30 insulin represented by the following formula (2), a recovery rate of human insulin represented by the following formula (1) is 70% or more when recovered in 99.5% purity:
(Liquid Chromatography Separation Conditions)

Column size: 150 mm×4.6 mm inner diameter
Eluent: water/pH adjusting agent/water-soluble organic solvent/salt Flow velocity: 1 mL/min
Column temperature: 25°C
Measurement wavelength: UV 280 nm
Sample: a 100 mg/mL solution prepared from an insulin mixture containing 96 mass% of human insulin represented by the following formula (1) and 4 mass% of desB30 insulin represented by the following formula (2)
Sample injection volume: 100 μL

[Chem. 1]

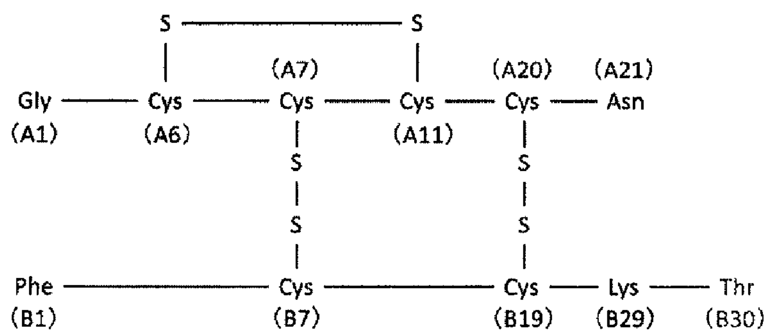

· · · (1)

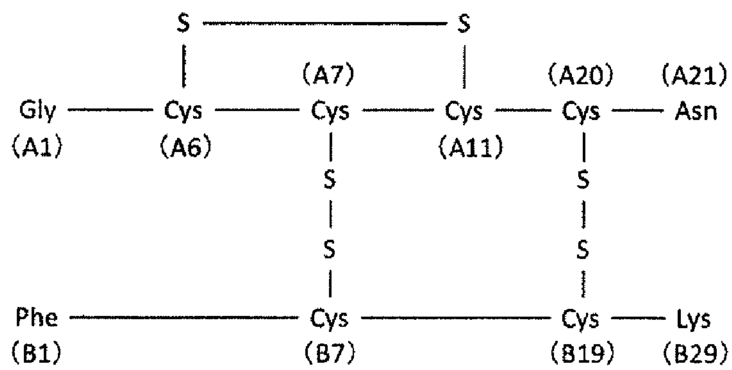

· · · (2)

2. The separating agent for purification of human insulin according to claim 1,
wherein a volume average particle diameter of the separating agent is from 1 to 50 μm.

3. The separating agent for purification of human insulin according to claim 1 or 2, comprising: porous particles; and an ion exchange group bonded to the porous particles.

4. The separating agent for purification of human insulin according to claim 3, wherein the ion exchange group contains a strongly acidic functional group.

5. The separating agent for purification of human insulin according to claim 4, wherein an amount of the introduced strongly acidic functional group is from 0.045 to 0.25 meq/g per unit mass in a wet state of the separating agent.

6. The separating agent for purification of human insulin according to any one of claims 3 to 5, wherein the ion exchange group is a water-soluble polymer containing an ionic functional group.

7. A separating agent for purification of human insulin, comprising:

porous particles; and
an ion exchange group bonded to the porous particles, wherein
the ion exchange group contains a strongly acidic functional group, and
an amount of the introduced strongly acidic functional group is from 0.045 to 0.25 meq/g per unit mass in a wet state of the separating agent.

8. A separating agent for purification of human insulin, wherein at the time of isolating human insulin represented by the following formula (1) under the following liquid chromatography separation conditions by using the separating agent from a solution containing human insulin represented by the following formula (1) and A21 desamidoinsulin represented by the following formula (3), a recovery rate of human insulin represented by the following formula (1) is 97% or more when recovered in 99.9% purity:
(Liquid Chromatography Separation Conditions)

Column size: 150 mm×4.6 mm inner diameter
Eluent: water/pH adjusting agent/water-soluble organic solvent
Flow velocity: 1 mL/min
Column temperature: 25°C
Measurement wavelength: UV 280 nm
Sample: a 100 mg/mL solution prepared from an insulin mixture containing 96 mass% of human insulin represented by the following formula (1) and 4 mass% of A21 desamidoinsulin represented by the following formula (3)
Sample injection volume: 400 μL

[Chem. 2]

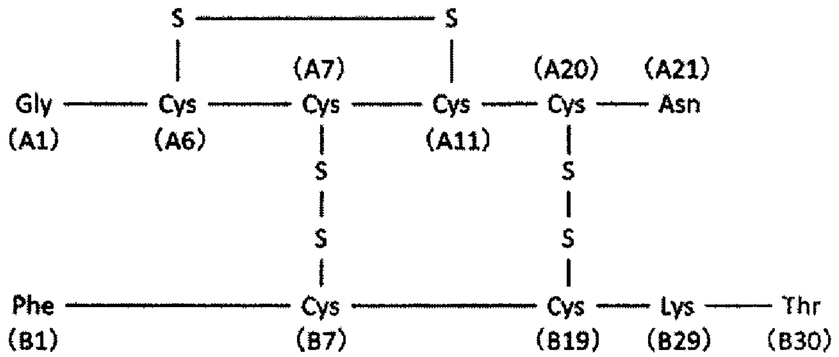

· · · (1)

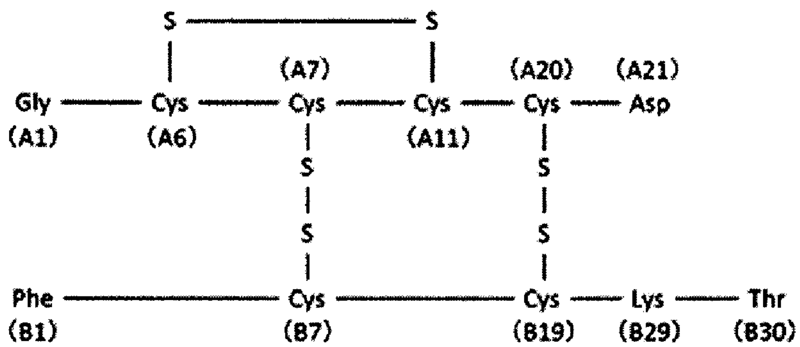

· · · (3)

.

**9.** The separating agent for purification of human insulin according to claim 8,
wherein a volume average particle diameter of the separating agent is from 1 to 50 μm.

**10.** The separating agent for purification of human insulin according to claim 8 or 9, comprising: porous particles; a hydrophobic functional group bonded to the porous particles; and an ion exchange group bonded to the porous particles.

**11.** The separating agent for purification of human insulin according to claim 10,
wherein the hydrophobic functional group is at least one selected from the group consisting of a saturated alkyl group, an unsaturated alkyl group, and an aryl group.

**12.** The separating agent for purification of human insulin according to claim 10 or 11,
wherein an amount of the introduced hydrophobic functional group is from 0.01 to 0.25 g/g per dry mass of the separating agent.

**13.** The separating agent for purification of human insulin according to any one of claims 10 to 12,
wherein the ion exchange group is an anion exchange group.

**14.** The separating agent for purification of human insulin according to any one of claims 10 to 13,
wherein an amount of the introduced ion exchange group is from 0.01 to 2.0 meq/g per dry mass of the separating agent.

**15.** A separating agent for purification of human insulin, comprising:

porous particles;
a hydrophobic functional group bonded to the porous particles; and
an ion exchange group bonded to the porous particles, wherein
the ion exchange group is an anion exchange group, and
an amount of the introduced ion exchange group is from 0.01 to 2.0 meq/g per dry mass of the separating agent.

**16.** A human insulin purification method using the separating agent for purification of human insulin according to any one of claims 1 to 15.

**INTERNATIONAL SEARCH REPORT**

International application No.
PCT/JP2018/004494

A. CLASSIFICATION OF SUBJECT MATTER
Int.Cl. C07K1/18(2006.01)i, B01D15/36(2006.01)i, B01J39/05(2017.01)i, B01J39/26(2006.01)i, B01J41/04(2017.01)i, B01J41/20(2006.01)i, C07K14/62(2006.01)i

According to International Patent Classification (IPC) or to both national classification and IPC

B. FIELDS SEARCHED

Minimum documentation searched (classification system followed by classification symbols)
Int.Cl. C07K1/18, B01D15/36, B01J39/05, B01J39/26, B01J41/04, B01J41/20, C07K14/62

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)
JSTPlus/JMEDPlus/JST7580 (JDreamIII), CAplus/MEDLINE/EMBASE/BIOSIS (STN), WPIDS/WPIX (STN)

C. DOCUMENTS CONSIDERED TO BE RELEVANT

| Category* | Citation of document, with indication, where appropriate, of the relevant passages | Relevant to claim No. |
|---|---|---|
| X<br>Y | JP 2014-530211 A (GE HEALTHCARE BIO-SCIENCES AB) 17 November 2014, claims, paragraphs [0003], [0017]-[0018], [0026]-[0030], [0038]-[0048], table 1 & US 2014/0243498 A1, claims, paragraphs [0003], [0022]-[0023], [0035]-[0043], [0057]-[0076], table 1 & EP 2748181 A1 & WO 2013/048330 A1 | 1-7, 16<br>1-16 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 24 April 2018 (24.04.2018) | 15 May 2018 (15.05.2018) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

INTERNATIONAL SEARCH REPORT

International application No.
PCT/JP2018/004494

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2008-232764 A (TOSOH CORP.) 02 October 2008, claims, examples 1, 3-6 (Family: none) | 1-7, 16 |
| Y | JP 10-182700 A (HOECHST AG.) 07 July 1998, claims & US 5977297 A1, claims & EP 849277 A3 | 1-16 |
| Y | JP 64-86896 A (HOECHST AG.) 31 March 1989, claims, examples & US 5101013 A1 claims, examples & EP 305760 A2 | 1-16 |
| Y | JP 2016-6410 A (JNC CORPORATION) 14 January 2016, claims, paragraph [0024], example 1 & US 2015/0344520 A1, claims, example [0059], example 1 | 8-16 |
| A | JP 2014-524916 A (INSTRACTION GMBH) 25 September 2014, entire text & US 2014/0336355 A1, entire text & EP 2731958 A1 & WO 2013/007799 A1 | 1-16 |
| A | JP 6-228205 A (MITSUBISHI KASEI CORP.) 16 August 1994, entire text (Family: none) | 1-16 |
| A | MOSLEMI, P., et al., "A rapid and sensitive method for simultaneous determination of insulin and A21-desamido insulin by high-performance liquid chromatography", Journal of Pharmaceutical and Biomedical Analysis (2003) vol. 33, pp. 45-51 ISSN:0731-7085 entire text | 1-16 |
| A | KROEFF, E. P., et al., "Production scale purification of biosynthetic human insulin by reversed-phase high-performance liquid chromatography", Journal of Chromatography (1989) vol. 461, pp. 45-61 ISSN:0021-9673 entire text | 1-16 |

Form PCT/ISA/210 (continuation of second sheet) (January 2015)

**INTERNATIONAL SEARCH REPORT**

International application No. PCT/JP2018/004494

**Box No. II Observations where certain claims were found unsearchable (Continuation of item 2 of first sheet)**

This international search report has not been established in respect of certain claims under Article 17(2)(a) for the following reasons:

1. ☐ Claims Nos.: because they relate to subject matter not required to be searched by this Authority, namely:

2. ☐ Claims Nos.: because they relate to parts of the international application that do not comply with the prescribed requirements to such an extent that no meaningful international search can be carried out, specifically:

3. ☐ Claims Nos.: because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
See extra sheet

1. ☐ As all required additional search fees were timely paid by the applicant, this international search report covers all searchable claims.
2. ☒ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (January 2015)

**INTERNATIONAL SEARCH REPORT**

International application No.
PCT/JP2018/004494

<Continuation of Box □>
(i) Number of inventions included in the claims
2
(ii) Claim numbers of claims classified into each invention
Invention 1: Claims 1-7, and 16 (part thereof)
Invention 2: Claims 8-15, and 16 (part thereof)
(iii) Reason for being judged not to comply with the requirements of unity of invention
(Invention 1) Claims 1-7, and 16

The invention in claims 1-6 and the part of the invention in claim 16 referring to claims 1-6 have the special technical feature in which "when a human insulin represented by formula (1) below is isolated, using said separating agent under a separation condition of liquid chromatography, from a solution including the human insulin represented by formula (1) below and a desB30 insulin represented by formula (2) below, the recovery rate of the human insulin represented by formula (1) below as recovered at 99.5% purity is 70% or higher", and thus are classified as invention 1. The invention in claim 7 and the part of the invention in claim 16 referring to claim 7 have a special technical feature corresponding to the invention in claim 1, and thus are classified as invention 1.

(Invention 2) Claims 8-16

The invention in claims 8-15 and the part of the invention in claim 16 referring to claims 8-15 do not have the special technical feature of the invention in claim 1. Meanwhile, the invention in claims 8-15 and the part of the invention in claim 16 referring to claims 8-15 have common or corresponding special technical features, and thus are classified as invention 2.

<Concerning the subject of the search>

The separating agent for purification of human insulin of the invention in claim 1 is only specified as having the characteristic in which "when a human insulin represented by formula (1) below is isolated, using said separating agent under a separation condition of liquid chromatography, from a solution including the human insulin represented by formula (1) below and a desB30 insulin represented by formula (2) below, the recovery rate of the human insulin represented by formula (1) below as recovered at 99.5% purity is 70% or higher".

However, in the specification, only a separating agent for purification of human insulin and including porous particles and ion exchange groups bonded to the porous particles, wherein the ion exchange groups include a strongly acidic functional group, and the introduced amount of the strongly acidic functional group is 0.045-0.25 meq/g per unit mass of said separating agent in a wet state, is disclosed as an article having said characteristic.

In addition, it was common technical knowledge at the time of filing that it is difficult to understand which specific separating agent has which specific capability in chromatography, and no basis can be found for expanding or generalizing the disclosure of the description to the scope of the invention in claim 1 solely on account of said characteristic.

Accordingly, the invention in claim 1 is beyond the scope of the disclosure of the description, and does not comply with the requirements for evidence under PCT Article 6.

In addition, considering the disclosure of the description as above and common technical knowledge at the time of filing, it is recognized that obtaining human insulin having said characteristic when carrying out the invention in claim 1 requires trial and error, involving the production and examination of a countless number of separating agents, beyond that which could be expected of a person skilled in the art.

Form PCT/ISA/210 (extra sheet) (January 2015)

**INTERNATIONAL SEARCH REPORT**

International application No.
PCT/JP2018/004494

Thus, the description is not clearly or sufficiently disclosed such that a person skilled in the art could carry out the invention in claim 1, and thus does not satisfy the requirements of PCT Article 5.

In addition, considering said common technical knowledge, since the shape of a substance contained in the separating agent, the functional group, or the like required in order for the separating agent to have said characteristic is not prescribed at all, it is clear that the "separating agent for purification of human insulin" is not sufficiently defined technically solely on account of said characteristic, and the invention cannot be clearly understood from the disclosure of claim 1, even when taking into account the description and the disclosure of the drawings.

Accordingly, claim 1 does not comply with the requirements for clarity under PCT Article 6. This is also true with respect to claims 2-4, 6, 8-14, and 16.

Accordingly, meaningful searches for parts other than the "separating agent for purification of human insulin and including porous particles and ion exchange groups bonded to the porous particles, wherein the ion exchange group includes a strongly acidic functional group, and the introduced amount of the strongly acidic functional group is 0.045-0.25 meq/g per unit mass of said separating agent in a wet state" in the invention in claims 1-7, and 16 of the present application, and parts other than the separating agent which is for purification of human insulin "and includes porous particles, hydrophobic functional groups bonded to the porous particles, and ion exchange groups bonded to the porous particles, wherein the ion exchange group is an anion exchange group, and the introduced amount of the strongly acidic functional group is 0.01-2.0 meq/g per dry mass of said separating agent in the invention in claims 8-16 cannot be carried out, and thus searches were not carried out for said parts.

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 10182700 A
- JP 63116916 A
- JP 1289802 A


### Non-patent literature cited in the description


- Kromasil (registered trademark) of Akzo Nobel Company, Homepage News. *Using EternityXT stationary phase for analysis and purification under reversed-phase chromatography,* May 2016, https://www.kromasil.com/notes/?archive